(19) **Europäisches Patentamt**
**European Patent Office**
**Office européen des brevets**

(11) **EP 4 652 957 A1**

(12) **EUROPEAN PATENT APPLICATION**

(43) Date of publication:
26.11.2025 Bulletin 2025/48

(21) Application number: 25177372.7

(22) Date of filing: 19.05.2025

(51) International Patent Classification (IPC):
*A61B 90/00* (2016.01)    *A61B 34/20* (2016.01)

(52) Cooperative Patent Classification (CPC):
**A61B 90/39; A61B 17/3403; G06N 3/02;**
**G06N 20/00;** A61B 34/20; A61B 2017/3413;
A61B 2034/2055; A61B 2034/2065; A61B 2090/364;
A61B 2090/3937; A61B 2090/3983

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: 21.05.2024 JP 2024082859
07.05.2025 JP 2025077371

(71) Applicant: **FUJIFILM Corporation**
**Tokyo 106-8620 (JP)**

(72) Inventors:
• **SASUGA, Saeko**
**Tokyo, 106-8620 (JP)**
• **ISHIDA, Koichi**
**Tokyo, 106-8620 (JP)**

(74) Representative: **Dehns Germany Partnerschaft**
**mbB**
**Theresienstraße 6-8**
**80333 München (DE)**

(54) **MEDICAL INSTRUMENT, MEDICAL SUPPORT DEVICE, AND MEDICAL SUPPORT PROGRAM**

(57)    A medical instrument including a first region provided with a first marker, an intermediate region not provided with a marker, and a second region provided with a second marker, which are disposed in a longitudinal direction, wherein: the first marker includes plural first pattern rows arranged along a direction that intersects with the longitudinal direction, the second marker includes plural second pattern rows arranged along the direction that intersects with the longitudinal direction, each pattern rows being configured by plural symbols arranged along the longitudinal direction, and an arrangement of the symbols in each pattern row being different from an arrangement of the symbols in any pattern row adjacent thereto.

FIG. 7

**Description**

BACKGROUND OF THE INVENTION

1. Field of the Invention

The present disclosure relates to a medical instrument, a medical support device, and a medical support program.

2. Description of the Related Art

In the related art, a technology for specifying a position and an orientation of a medical instrument inserted into a body is known. For example, JP2018-94020A discloses that a position of an ultrasound probe in a surgical field is acquired by a magnetic sensor. Note that such a method using a magnetic sensor may be unsuitable for use because it may be influenced by or may affect a metallic medical instrument other than the ultrasound probe.

Therefore, a method that does not use a magnetic sensor has also been proposed. For example, WO2023/162657A discloses a method of acquiring a surgical field image by optically imaging an ultrasound probe provided with a marker using an endoscope, and estimating a position and an orientation of the ultrasound probe by detecting the marker from the surgical field image. In addition, for example, JP2017-501802A discloses a method of providing a marker on an ophthalmic surgical instrument to be inserted into an eyeball, and determining a position and an orientation of the ophthalmic surgical instrument by detecting the marker from a fundus image.

SUMMARY OF THE INVENTION

Due to the characteristics of the medical instrument to be inserted into the body, it may not be possible to avoid a situation in which it is difficult to optically detect the marker for specifying the position and the orientation of the medical instrument. For example, the marker may be shielded by an organ or the like. In addition, for example, in a case in which illumination light for capturing an optical image in the body is reflected on a surface of the marker (medical instrument), a marker portion may appear overexposed in the optical image. In addition, for example, depending on a positional relationship between the marker and a camera, the marker may not be included in the angle of view of the optical image.

In such a situation, moving the medical instrument to a position and an orientation where the marker can be detected is not preferable from the viewpoint of efficiency and low invasiveness. In the technology in the related art in which the marker is optically detected, in a case in which the marker cannot be detected even partially, the accuracy of specifying the position and the orientation of the medical instrument is lowered or cannot be specified, and thus the practicality is insufficient.

The present disclosure provides a medical instrument, a medical support device, and a medical support program capable of specifying a position and an orientation of a medical instrument with high accuracy.

According to a first aspect of the present disclosure, there is provided a medical instrument including: a first region provided with a first marker, an intermediate region not provided with a marker, and a second region provided with a second marker, which are disposed in a longitudinal direction, in which the first marker includes plural first pattern rows arranged along a direction that intersects with the longitudinal direction, each of the first pattern rows being configured by plural symbols arranged along the longitudinal direction, an arrangement of the symbols in each first pattern row being different from an arrangement of the symbols in any first pattern row adjacent thereto, and the second marker includes plural second pattern rows arranged along the direction that intersects with the longitudinal direction, each of the second pattern rows being configured by plural symbols arranged along the longitudinal direction, an arrangement of the symbols in each second pattern row being different from an arrangement of the symbols in any second pattern row adjacent thereto.

In the above aspect, at least a part of a surface of the first region may be formed as a curved surface, and at least a part of a surface of the second region may be formed as a curved surface.

In the above aspect, at least one of the first marker or the second marker may include a pattern row composed of an arrangement of the symbols not included in the other.

In the above aspect, the plurality of first pattern rows and the plurality of second pattern rows may all have different arrangements of the symbols.

In the above aspect, at least one of the first marker or the second marker may include at least one pattern row including the symbol of a type not included in the other.

In the above aspect, in a case in which a direction from the first marker to the second marker is defined as a forward direction and a direction from the second marker to the first marker is defined as a reverse direction, an arrangement of the symbols along the forward direction in at least one of the first pattern rows may be the same as an arrangement of the symbols along the reverse direction in any one of the other first pattern rows, and an arrangement of the symbols along the forward direction in at least one of the second pattern rows may be the same as an arrangement of the symbols along the reverse direction in any one of the other second pattern rows.

In the above aspect, in the first marker, the plurality of first pattern rows may be arranged such that only a combination of the first pattern rows having a Hamming distance of 2 or more is included in a predetermined range in the direction intersecting the longitudinal direction, and in the second marker, the plurality of second pattern rows may be arranged such that only a combination of the second pattern rows having a Hamming distance of 2 or more is included in a predetermined range in the direction intersecting the longitudinal direction.

In the above aspect, types of the symbols constituting the first pattern row and types of the symbols constituting the second pattern row may be the same.

In the above aspect, colors of the symbols constituting the first pattern row and colors of the symbols constituting the second pattern row may all be the same.

In the above aspect, the number of the symbols constituting the first pattern row and the number of the symbols constituting the second pattern row may be the same.

In the above aspect, the first pattern row may be configured by arranging a total of four symbols formed of two types of the symbols having different shapes, and the second pattern row may be configured by arranging a total of four symbols formed of the same two types of the symbols as the types of the symbols included in the first pattern row.

In the above aspect, the intermediate region may be provided with information other than the marker.

In the above aspect, the intermediate region may be provided with information indicating a predetermined direction.

In the above aspect, the intermediate region may be provided with gradations.

In the above aspect, the intermediate region may be further provided with information indicating a center of the gradations.

In the above aspect, the medical instrument may be a medical probe capable of observing an internal structure of an organ.

According to a second aspect of the present disclosure, there is provided a medical support device comprising: a processor, in which the medical support device specifies a position and an orientation of the medical instrument according to the first aspect, and the processor is configured to acquire an optical image by optically imaging the medical instrument inserted into the body with a camera, extract at least a part of pattern rows, from among the plurality of first pattern rows and the plurality of second pattern rows, from the optical image, and specify the position and the orientation of the medical instrument based on at least the part of the pattern rows that is extracted.

In the second aspect, the processor may be configured to specify a positional relationship between the first marker and the second marker based on a shape of the medical instrument included in the optical image, and specify the position and the orientation of the medical instrument based on at least the part of the pattern rows that is extracted and the specified positional relationship between the first marker and the second marker.

In the second aspect, the processor may be configured to detect the first region and the second region from the optical image using a trained model that has been trained in advance by using the optical image as an input and the first region and the second region in the input optical image as an output, specify a positional relationship between the first marker and the second marker based on the detected first region and the detected second region, and specify the position and the orientation of the medical instrument based on at least the part of the pattern rows that is extracted and the specified positional relationship between the first marker and the second marker.

According to a third aspect of the present disclosure, there is provided a medical support program for causing a computer to execute a process of specifying a position and an orientation of the medical instrument according to the first aspect, the process comprising: acquiring an optical image by optically imaging the medical instrument inserted into the body with a camera; extracting at least a part of pattern rows, from among the plurality of first pattern rows and the plurality of second pattern rows, from the optical image; and specifying the position and the orientation of the medical instrument based on at least the part of the pattern rows that is extracted.

According to the above aspects, the medical instrument, the medical support device, and the medical support program of the present disclosure can specify the position and the orientation of the medical instrument with high accuracy.

BRIEF DESCRIPTION OF THE DRAWINGS

Fig. 1 is a diagram showing an outline of a medical support system including a medical support device.
Fig. 2 is a diagram showing a state of an inside of a body in an endoscopic surgery.
Fig. 3 is an explanatory diagram of a first guide groove of an ultrasound probe.
Fig. 4 is an explanatory diagram of a second guide groove of the ultrasound probe.
Fig. 5 is a diagram showing an insertion state of a biopsy needle guided by the first guide groove.

Fig. 6 is a diagram showing an insertion state of the biopsy needle guided by the second guide groove.
Fig. 7 is a diagram showing an example of a configuration of a marker.
Fig. 8 is a diagram showing another example of a configuration of a marker.
Fig. 9 is a diagram showing a hardware configuration of the medical support device.
Fig. 10 is a diagram showing a relationship in a position and an orientation between the marker and the ultrasound probe.
Fig. 11 is another diagram showing a relationship in a position and an orientation between the marker and the ultrasound probe.
Fig. 12 is a flowchart showing an example of medical support processing.
Fig. 13 is a diagram showing another example of a symbol.
Fig. 14 is a diagram showing another example of a symbol.
Fig. 15 is a diagram showing another example of a symbol.
Fig. 16 is a diagram showing another example of a symbol.
Fig. 17 is a diagram showing another example of support information.
Fig. 18 is a diagram showing another example of support information.
Fig. 19 is a diagram showing another example of a configuration of a marker.

DESCRIPTION OF THE PREFERRED EMBODIMENTS

Hereinafter, an example of an embodiment of the disclosed technology will be described with reference to the drawings. The same or equivalent components and parts in the respective drawings are denoted by the same reference numerals, and the duplicated description will be omitted. In addition, dimensional ratios in the drawings are exaggerated for convenience of description and may be different from the actual ratios.

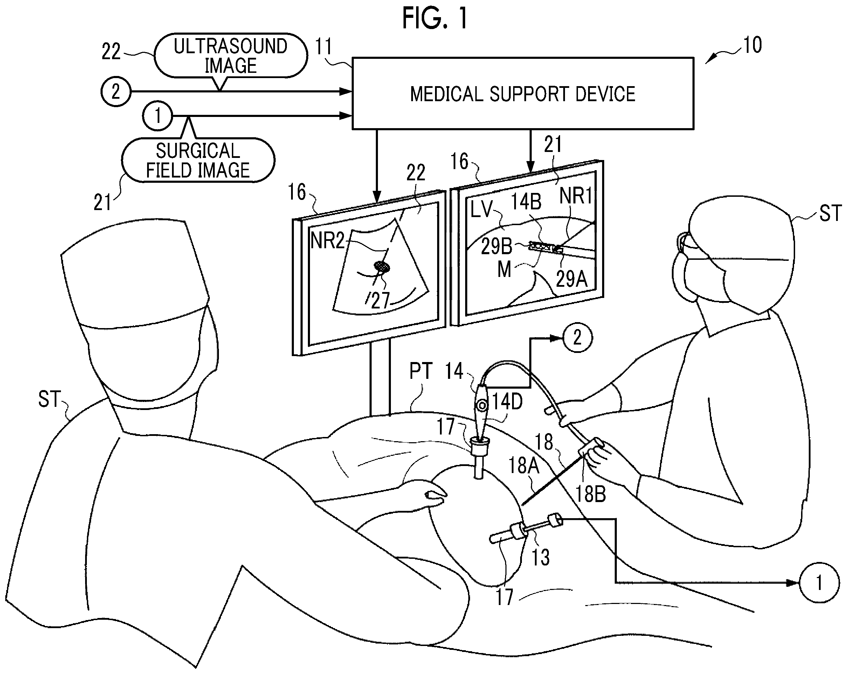

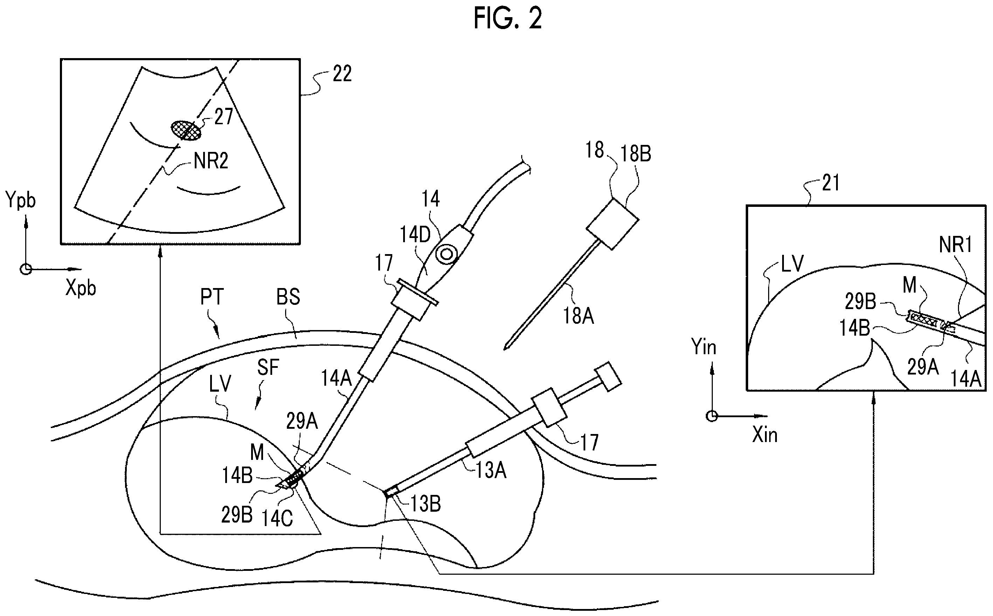

As shown in Figs. 1 and 2, a medical support system 10 is used, for example, in a case in which an endoscopic surgery using an endoscope 13 is performed on a patient PT. The endoscopic surgery is a surgery that is performed by making a small hole in a body of the patient PT and inserting a medical instrument such as the endoscope 13 through the hole, unlike a laparotomy. The medical support system 10 provides a medical staff ST including a doctor with a visual field of a surgical field inside the body of the patient PT, and with support information for supporting medical care such as a surgery and an examination. As will be described below, the support information is, specifically, a puncture path of a biopsy needle 18 and the like.

Such a medical support system 10 has a function of providing the support information in real time during a surgery, and is therefore also called a surgical navigation system or the like.

As shown in Fig. 1, the medical support system 10 comprises, for example, a medical support device 11, an endoscope 13, an ultrasound probe 14, and a display 16. The medical support device 11 is communicably connected to the endoscope 13, the ultrasound probe 14, and the display 16.

Fig. 2 shows a state in which the endoscope 13 and the ultrasound probe 14 are inserted into the abdomen of the patient PT. In the endoscopic surgery, a part of the endoscope 13 and a part of the ultrasound probe 14 including distal end parts thereof are inserted into the body via a trocar 17. The trocar 17 is an insertion tool having an insertion hole into which the endoscope 13 or the like is inserted and a valve provided in the insertion hole to prevent gas leakage. In the endoscopic surgery, the trocar 17 is used for insertion into the body, such as the endoscope 13 and the ultrasound probe 14, because pneumoper itoneum is performed by injecting carbon dioxide gas into an abdominal cavity.

The biopsy needle 18 is a treatment tool for puncturing a lesion such as a tumor included in an organ as a puncture target.

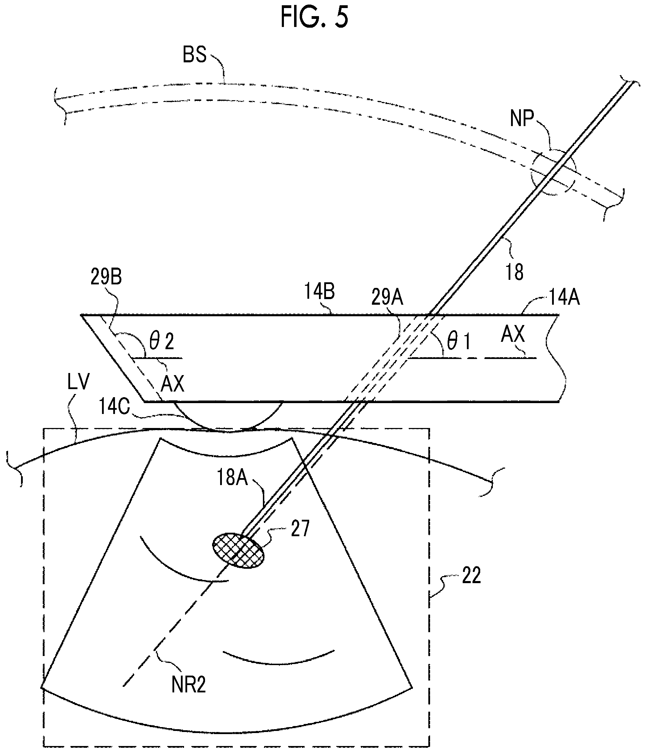

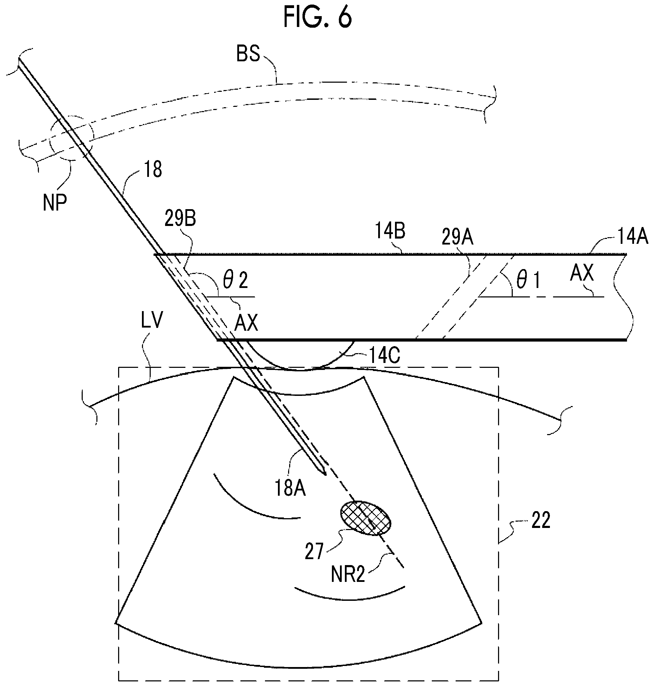

The biopsy needle 18 is, for example, a cauterization biopsy needle used for cauterization of the lesion. The cauterization biopsy needle has an electrode at its distal end to which a high-frequency voltage is applied, and, in a case in which the high-frequency voltage is applied in a state in which the lesion is punctured by the electrode, the lesion is necrotized by heat generated by the electrode. In this example, as the surgery, an example will be described in which a treatment of necrotizing a tumor 27 of a liver LV is performed by visualizing the tumor 27 by using an ultrasound image 22 and cauterizing the visualized tumor 27 by using the biopsy needle 18 (see also Figs. 5 and 6). The biopsy needle 18 has a needle part 18A and a grip part 18B provided on a base end side of the needle part 18A.

The endoscope 13 has an insertion part 13A to be inserted into the body of the patient PT. A camera 13B and a light source (for example, a light emitting diode (LED)) for illumination are incorporated in a distal end part of the insertion part 13A. The endoscope 13 is, for example, a rigid endoscope in which the insertion part 13A is rigid, and is often used for abdominal cavity observation, so that the endoscope 13 is also called a laparoscope. The camera 13B has an image sensor such as a charge coupled device (CCD) image sensor and a complementary metal oxide semiconductor (CMOS) image sensor, and an imaging optical system including a lens that forms a subject image on an imaging surface of the image sensor. The image sensor is, for example, an image sensor capable of capturing a color image.

The endoscope 13 optically images a surgical field SF including a target part (in this example, the liver LV) inside the body of the patient PT by using the camera 13B. The surgical field SF is a space that spreads in a body cavity defined by an organ and a body wall inside the body. The endoscope 13 is connected to an image processing processor for an endoscope (not shown), and the image processing processor performs signal processing on an imaging signal output from the image sensor to generate a surgical field image 21 of the surgical field SF inside the body. The camera 13B is an example of a "camera" of the present disclosure. The surgical field image 21 is an example of an "optical image" of the present disclosure.

Visible light such as white light is used as illumination light for the endoscope 13. As the illumination light of the endoscope 13, special light such as ultraviolet light and infrared light may be used. As the special light, light restricted to a specific wavelength such as short-wavelength narrow-band light obtained by narrowing down light in a short wavelength range such as an ultraviolet range may be used. The surgical field image 21 captured by the endoscope 13 is transmitted to the medical support device 11 in real time via the image processing processor for an endoscope. In Fig. 2, reference numerals Xin and Yin indicate a coordinate system of the surgical field image 21.

The ultrasound probe 14 has an insertion part 14A to be inserted into the body of the patient PT and an operation part 14D on a base end side of the insertion part 14A. An ultrasound transducer 14C is incorporated in a distal end part 14B of the insertion part 14A. The ultrasound transducer 14C transmits an ultrasound wave to the target part and receives a reflected wave reflected by the target part. The ultrasound probe 14 is connected to an image processing processor for an ultrasound probe (not shown).

The image processing processor for an ultrasound probe performs image reconstruction processing based on the reflected wave based on a signal corresponding to the reflected wave received by the ultrasound probe 14. Through the image reconstruction processing, the ultrasound image 22 showing an internal structure of the target part scanned by the ultrasound probe 14 is generated. The ultrasound image 22 is a so-called brightness (B)-mode image in which an internal structure from a surface layer to a deep layer where the ultrasound wave reaches the target part is visualized as brightness information. The ultrasound image 22 visualizes an internal structure of the target part that cannot be observed in the surgical field image 21 obtained by optical imaging. The ultrasound probe 14 is an example of a "medical instrument" and a "medical probe capable of observing an internal structure of an organ" of the present disclosure.

The ultrasound probe 14 is, for example, a convex type that radially transmits ultrasound waves, and acquires a fan-shaped image with the ultrasound transducer 14C as a base point. A plurality of the ultrasound images 22 are captured along a scanning direction by performing the scanning with the ultrasound probe 14. The ultrasound image 22 is transmitted to the medical support device 11 in real time via the image processing processor for an ultrasound probe. In Fig. 2, reference numerals Xpb and Ypb indicate a coordinate system of the ultrasound image 22.

A guide groove 29 is provided in the distal end part 14B of the insertion part 14A. The guide groove 29 is a guide groove for engaging with the biopsy needle 18 to guide the insertion of the biopsy needle 18 to a target position inside the organ. In this example, as the guide groove 29, two guide grooves, that is, a first guide groove 29A and a second guide groove 29B, are provided. Hereinafter, in a case in which there is no need to distinguish between the first guide groove 29A and the second guide groove 29B, both will be collectively referred to as the guide groove 29.

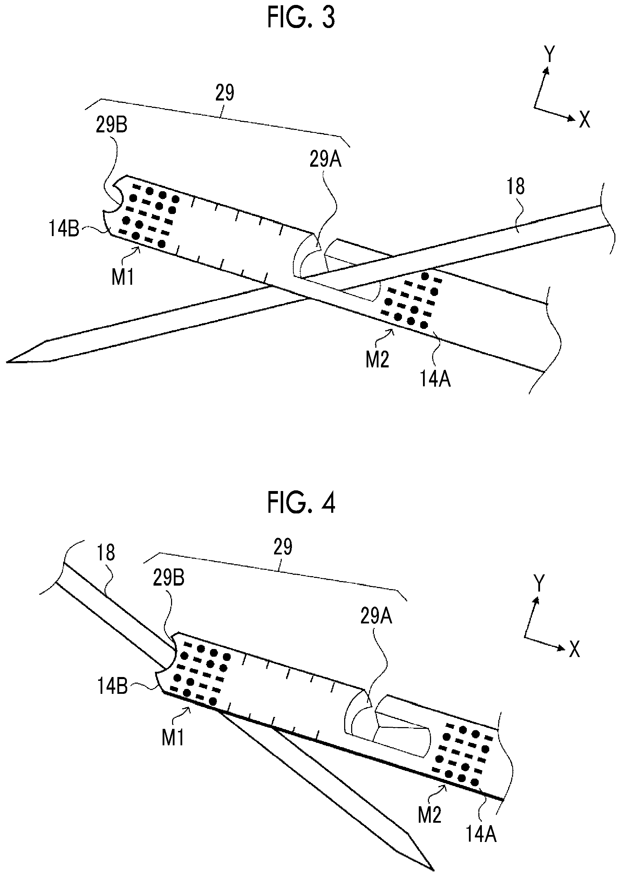

Fig. 3 shows a state of the biopsy needle 18 guided by the first guide groove 29A. Fig. 4 shows a state of the biopsy needle 18 guided by the second guide groove 29B. Fig. 5 schematically shows a state in which the biopsy needle 18 inserted into the body is guided by the first guide groove 29A and the tumor 27 in the liver LV is punctured by the biopsy needle 18. Fig. 6 schematically shows a state in which the biopsy needle 18 inserted into the body is guided by the second guide groove 29B and the tumor 27 in the liver LV is punctured by the biopsy needle 18.

The first guide groove 29A is the guide groove 29 provided on the base end side with respect to the ultrasound transducer 14C in the distal end part 14B. The first guide groove 29A is inclined at an angle of θ1 with respect to a direction of an axis AX of the distal end part 14B. The first guide groove 29A is inclined rearward such that a needle tip of the biopsy needle 18 to be inserted from the base end side of the distal end part 14B is directed toward the distal end side of the distal end part 14B.

The second guide groove 29B is the guide groove 29 formed at the most distal end of the distal end part 14B and provided on the distal end side with respect to the ultrasound transducer 14C. The second guide groove 29B is inclined at an angle of θ2 with respect to a direction of an axis AX of the distal end part 14B. The second guide groove 29B is inclined rearward such that the needle tip of the biopsy needle 18 to be inserted from the distal end side of the distal end part 14B is directed toward the base end side of the distal end part 14B.

The biopsy needle 18 is inserted while the tumor 27 is checked by the ultrasound image 22. Since the first guide groove 29A and the second guide groove 29B are inclined toward the ultrasound transducer 14C, the needle tip of the biopsy needle 18 can be directed toward the region visualized by the ultrasound image 22.

As shown in Figs. 3 and 4, the distal end part 14B of the insertion part 14A is provided with a first marker M1 and a second marker M2. Hereinafter, in a case in which there is no need to distinguish between the first marker M1 and the second marker M2, both will be collectively referred to as a marker M. A configuration of the marker M will be described below. In order to avoid complication of the drawings, the marker M is simplified or omitted in some drawings.

The marker M is a marker that is recognizable from the surgical field image 21 optically captured by the camera 13B of the endoscope 13, that is, an optically detectable marker. The marker M is used by the medical support device 11 to specify a position and an orientation of the ultrasound probe 14, more specifically, a position and an orientation of the distal end part 14B of the insertion part 14A. A method of specifying the position and the orientation of the ultrasound probe 14 using the marker M will be described below.

The medical support device 11 acquires the surgical field image 21 from the endoscope 13, and acquires the ultrasound image 22 from the ultrasound probe 14. As shown in Fig. 1, two displays 16 are provided as an example, and two images of the surgical field image 21 and the ultrasound image 22 are displayed on the respective displays 16. The surgical field image 21 and the ultrasound image 22 are output as a video, and are displayed on the displays 16 as a live view.

In a case in which the ultrasound probe 14 is inserted into the surgical field SF, the ultrasound probe 14, more specifically, the distal end part 14B of the insertion part 14A is reflected in the surgical field image 21. The medical support device 11 outputs the surgical field image 21 in which the ultrasound probe 14 is reflected to the display 16. A visual field of the surgical field SF inside the body of the patient PT is provided to the medical staff ST through a screen of the display 16.

As shown in Figs. 5 and 6, a second puncture line NR2 indicating a path passing through the first guide groove 29A or the second guide groove 29B for guiding the biopsy needle 18 can be superimposed and displayed on the ultrasound image 22. There are two types of second puncture lines NR2, one of which indicates a puncture path along an inclination angle $\theta 1$ of the first guide groove 29A and the other of which indicates a puncture path along an inclination angle $\theta 2$ of the second guide groove 29B. The second puncture line NR2 is generated as an extension line of the guide groove 29 based on a known positional relationship between the ultrasound transducer 14C and the guide groove 29.

The second puncture line NR2 is used as a guide in a case in which the target position is punctured by the biopsy needle 18. For example, in a case in which the target position punctured by the biopsy needle 18 is the tumor 27 in the liver LV, the ultrasound probe 14 is positioned by the medical staff ST such that the second puncture line NR2 and the tumor 27 overlap in the ultrasound image 22. In this state, the tumor 27 is punctured by the biopsy needle 18 with the second puncture line NR2 as a guide. In this way, in a state in which the position of the ultrasound probe 14 is adjusted such that the second puncture line NR2 passes through the target position, the biopsy needle 18 can reach the target position by allowing the biopsy needle 18 to pass through the guide groove 29.

However, since the ultrasound image 22 is an internal image of the organ, simply superimposing and displaying the second puncture line NR2 on the ultrasound image 22 makes it difficult to understand at which position and in what orientation on a body surface BS the biopsy needle 18 should be inserted in a case of inserting the biopsy needle 18 into the body from outside the body. Specifically, as shown in Figs. 5 and 6, it is difficult to understand an insertion angle of the biopsy needle 18 from the body surface BS toward the organ and an insertion position NP of the biopsy needle 18 on the body surface BS of the patient PT.

Therefore, the medical support device 11 superimposes and displays a first puncture line NR1 on the surgical field image 21 in addition to superimposing and displaying the second puncture line NR2 on the ultrasound image 22. The first puncture line NR1 indicates a puncture path from the body surface BS to the guide groove 29 of the ultrasound probe 14 in the surgical field image 21. The second puncture line NR2 indicates a puncture path from the guide groove 29 to a target position in an organ, such as the tumor 27 of the liver LV, in the ultrasound image 22. As a result, the medical staff ST is provided with the support information serving as a guide for the insertion angle and the insertion position NP of the biopsy needle 18.

In addition, in order to provide the support information, the medical support device 11 uses the marker M to specify the position and the orientation of the ultrasound probe 14, more specifically, the position and the orientation of the distal end part 14B of the insertion part 14A. The orientation of the distal end part 14B is represented by, for example, the direction of the axis AX of the distal end part 14B. Then, the position and the orientation of the guide groove 29 of which a relative positional relationship with the distal end part 14B is known are specified based on the specified direction of the axis AX of the distal end part 14B.

Configuration of Marker M

Incidentally, due to the characteristic of the ultrasound probe 14 being a medical instrument to be inserted into the body, it may not be possible to avoid a situation in which the marker M is difficult to optically detect. For example, the marker M may be shielded by an organ or the like. In addition, for example, in a case in which illumination light for capturing the surgical field image 21 in the body is reflected on a surface of the marker M, a portion of the marker M may appear overexposed in the surgical field image 21. In addition, for example, depending on a positional relationship between the marker M and the camera 13B, the entire marker M may not be included in the angle of view of the surgical field image 21.

In such a situation, moving the endoscope 13, the ultrasound probe 14, and the like to a position and an orientation where the entire marker M can be detected is not preferable from the viewpoint of efficiency and low invasiveness. Therefore, the marker M of the present disclosure is a so-called marker M having high redundancy, which can specify the position and the orientation of the ultrasound probe 14 with high accuracy even in a case in which a part of the marker M cannot be detected.

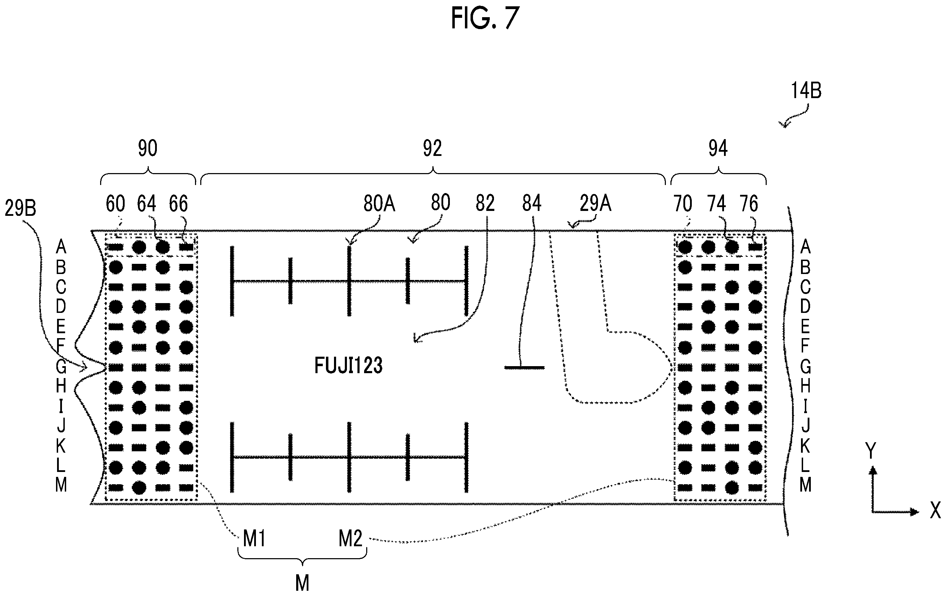

A configuration of the marker M will be described with reference to Fig. 7. Fig. 7 is an example of a developed view of a surface of the distal end part 14B. The distal end part 14B of the insertion part 14A includes, along a longitudinal direction, a first region 90 provided with a first marker M1, an intermediate region 92 provided with no marker M, and a second region 94 provided with a second marker M2. The term "longitudinal direction" refers to the direction of the axis AX of the distal end part 14B (X-axis direction in Fig. 7). In Fig. 7, a left side of a paper surface is the distal end side, and a right side of the paper surface is a base end side.

The first marker M1 includes plural first pattern rows 60 arranged along a direction that intersects with the longitudinal direction. Each of the first pattern rows 60 is configured by plural symbols 64 and 66 arranged along the longitudinal direction, and an arrangement of the symbols 64 and 66 in each first pattern row 60 differs from an arrangement of the symbols 64 and 66 in any first pattern row 60 adjacent thereto. The phrase "direction intersecting the longitudinal direction" refers to a direction (Y-axis direction in Fig. 7) intersecting the axis AX on the surface of the distal end part 14B, and is a circumferential direction of the ultrasound probe 14.

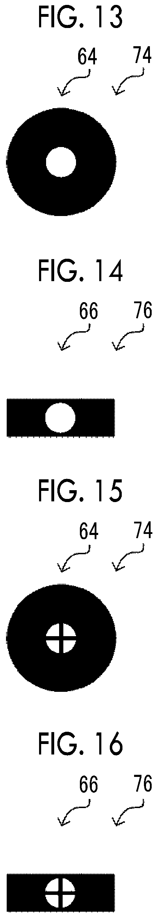

As an example, in Fig. 7, 13 first pattern rows 60 are arranged along the circumferential direction. Hereinafter, in a case of distinguishing between the 13 first pattern rows 60, reference numerals A to M are assigned in order from the first pattern row 60 at an upper end on the paper surface in Fig. 7, and are added to the end of the reference numerals. For example, the first pattern row 60 (the first pattern row 60 surrounded by a two-dot chain line) at the upper end on the paper surface in Fig. 7 on the paper surface is referred to as a "first pattern row 60A".

In addition, in the example of Fig. 7, the first pattern row 60 is configured by arranging a total of four symbols formed of two types of the symbols 64 and 66 having different shapes. For example, the first pattern row 60A is composed of a rectangular symbol 66, a circular symbol 64, a circular symbol 64, and a rectangular symbol 66 along the longitudinal direction. Among the other first pattern rows 60B to 60M, there is no one having the same arrangement as the first pattern row 60A. The 13 first pattern rows 60A to 60M have mutually different arrangements of the symbols 64 and 66.

The second marker M2 includes plural second pattern rows 70 arranged along the direction that intersects with the longitudinal direction. Each of the second pattern rows 70 is configured by plural symbols 74 and 76 arranged along the longitudinal direction, and an arrangement of the symbols 74 and 76 in each second pattern row 70 differs from an arrangement of the symbols 74 and 76 in any second pattern row 70 adjacent thereto.

As an example, in Fig. 7, 13 second pattern rows 70 are arranged along the circumferential direction. As with the first pattern row 60, in a case of distinguishing between the 13 second pattern rows 70, the 13 second pattern rows 70 will be referred to as second pattern rows 70A to 70M. The arrangement of the first pattern rows 60A to 60M and the arrangement of the second pattern rows 70A to 70M may overlap each other.

In addition, in the example of Fig. 7, the second pattern row 70 is configured by arranging a total of four symbols formed of the same two types of the symbols 74 and 76 as the types of the symbols 64 and 66 included in the first pattern row 60. For example, the second pattern row 70A is composed of a circular symbol 74, a circular symbol 74, a circular symbol 74, and a rectangular symbol 76 along the longitudinal direction. Among the other second pattern rows 70B to 70M, there is no one having the same arrangement as the second pattern row 70A. The 13 second pattern rows 70A to 70M have mutually different arrangements of the symbols 74 and 76.

A method of providing each of the first pattern row 60 and the second pattern row 70 to the ultrasound probe 14 is known. Therefore, in a case in which at least a part of pattern rows among the plurality of first pattern rows 60A to 60M and the plurality of second pattern rows 70A to 70M can be detected, the position and the orientation of the ultrasound probe 14 in the surgical field image 21 can be specified based on the detected pattern row and the known positional relationship between the ultrasound probe 14 and the pattern row.

In this way, the first marker M1 and the second marker M2, each of which can specify the position and the orientation of the ultrasound probe 14 by itself, are disposed apart from each other in the longitudinal direction with the intermediate region 92 interposed therebetween, so that the redundancy in the longitudinal direction can be ensured. For example, even in a situation in which only the first marker M1 is reflected in the surgical field image 21 and the second marker M2 is not reflected in the surgical field image 21, the position and the orientation of the ultrasound probe 14 can be specified based on the first marker M1 .

In addition, by configuring the first marker M1 and the second marker M2 with a plurality of pattern rows arranged in the circumferential direction, the redundancy in the circumferential direction can be ensured. For example, even in a situation in which streak-like overexposure occurs in the longitudinal direction due to the reflection of the illumination light for the endoscope 13, causing each pattern row that should be reflected in the surgical field image 21 to be missing, the position and the orientation of the ultrasound probe 14 can be specified based on the other pattern rows.

In this way, since the marker M according to the present embodiment ensures the redundancy in both the longitudinal direction and the circumferential direction, the position and the orientation of the ultrasound probe 14 can be specified with high accuracy in various situations, thereby improving the practicality. In a case in which both the first marker M1 and the second marker M2 can be detected, the position and the orientation of the entire ultrasound probe 14 can be specified from the positional relationship between the first marker M1 and the second marker M2, thereby further improving the specification accuracy.

As shown in Fig. 7, the plural first pattern rows 60 may have mutually different arrangements of symbols 64 and 66. With this configuration, the position and the orientation of the ultrasound probe 14 can be specified as long as even one of the first pattern rows 60 contained in the first marker M1 can be detected. Therefore, it is easy to avoid the inability to specify the position and the orientation. Likewise, the plural second pattern rows 70 may have mutually different arrangements of symbols 74 and 76, and the effect in this case is also the same as that of the first marker M1 .

Preferred Configuration and Possible Configuration of Marker M

The configuration of the marker M described above is a basic configuration of the marker M according to the present embodiment. Hereinafter, a preferred configuration and a possible configuration of the marker M will be described.

It is preferable that at least a part of a surface of the first region 90 is formed as a curved surface. Similarly, it is preferable that at least a part of a surface of the second region 94 is formed as a curved surface. By making a surface provided with the first marker M1 and the second marker M2 a curved surface, even in a case in which the reflection of the illumination light for the endoscope 13 occurs, it is possible to avoid the entire surface from being overexposed. Specifically, in the case of the curved surface, even in a case in which the reflection occurs, it will only result in streak-like overexposure. Therefore, the position and the orientation of the ultrasound probe 14 can be specified with high accuracy from a pattern row of a portion that is not overexposed.

The surfaces of the first region 90 and the second region 94 are not limited to being entirely curved surfaces, and may be formed of, for example, a combination of a curved surface and a flat surface. That is, at least a part of the first marker M1 and/or the second marker M2 need only be provided on a curved surface, and the first marker M1 and/or the second marker M2 may have a portion provided on a flat surface.

It is preferable that at least one of the first marker M1 or the second marker M2 includes a pattern row composed of an arrangement of the symbols not included in the other. Specifically, the first marker M1 and the second marker M2 may each include a unique arrangement pattern row. In addition, only one of the first marker M1 or the second marker M2 may include a unique arrangement pattern row.

For example, in Fig. 7, the first pattern row 60D included in the first marker M1 has a unique arrangement that does not overlap with any of the plurality of second pattern rows 70A to 70M included in the second marker M2. In addition, for example, the second pattern row 70B included in the second marker M2 has a unique arrangement that does not overlap with any of the plurality of first pattern rows 60A to 60M included in the first marker M1 .

Since at least one of the first marker M1 or the second marker M2 includes a unique pattern row, in a case in which the unique pattern row is detected from the surgical field image 21, it is possible to identify whether the marker M as the detection source is the first marker M1 or the second marker M2. For example, in a case in which any one of the first marker M1 or the second marker M2 is reflected in the surgical field image 21, it is possible to identify which one is reflected. In addition, for example, in a case in which both the first marker M1 and the second marker M2 are reflected in the surgical field image 21, it is possible to identify which is the first marker M1 and which is the second marker M2. Therefore, the position and the orientation of the entire ultrasound probe 14 can be specified from the relative positional relationship between the first marker M1 and the second marker M2, thereby further improving the specification accuracy.

The plurality of first pattern rows 60 and the plurality of second pattern rows 70 may all have different arrangements of the symbols. As described above, the method of providing each of the first pattern row 60 and the second pattern row 70 to the ultrasound probe 14 is known. Therefore, in a case in which the first pattern row 60 and the second pattern row 70 are configured not to overlap each other in arrangement, the position and the orientation of the ultrasound probe 14 can be specified as long as even a part of the pattern rows included in the first marker M1 or the second marker M2 can be detected. Therefore, it is easy to avoid the inability to specify the position and the orientation.

Fig. 7 illustrates an example in which the types of the symbols 64 and 66 constituting the first pattern row 60 and the types of the symbols 74 and 76 constituting the second pattern row 70 are the same, but the present disclosure is not limited to this.

At least one of the first marker M1 or the second marker M2 may include at least one pattern row including the symbol of a type not included in the other.

Specifically, both the first pattern row 60 and the second pattern row 70 may include a unique symbol. For example, the first pattern row 60 may be composed of a black circle symbol and a white circle symbol, and the second pattern row 70 may be composed of a rectangular symbol and a triangular symbol.

In addition, both the first pattern row 60 and the second pattern row 70 may include a common symbol and a unique symbol. For example, the first pattern row 60 may be composed of a black circle symbol and a white circle symbol, and the second pattern row 70 may be composed of a black circle symbol (that is, a common symbol) and a rectangular symbol (that is, a unique symbol).

In addition, any one of the first pattern row 60 or the second pattern row 70 may include a unique symbol in a part thereof. For example, the first pattern row 60 may be composed of two types of symbols of a black circle symbol and a white circle symbol, and the second pattern row 70 may be composed of three types of symbols of a black circle symbol (that is, a common symbol), a white circle symbol (that is, a common symbol), and a rectangular symbol (that is, a unique symbol). In this case, at least one of the plurality of second pattern rows 70 need only include a unique symbol, and the other second pattern rows 70 does not need to include the unique symbol.

Since at least one of the first marker M1 or the second marker M2 includes a unique symbol, in a case in which the unique symbol is detected from the surgical field image 21, it is possible to identify the first marker M1 and the second marker M2. As a result, the position and the orientation of the entire ultrasound probe 14 can be specified from the relative positional relationship between the first marker M1 and the second marker M2 in the surgical field image 21, thereby further improving the specification accuracy.

As described above, the plurality of first pattern rows 60 may have different arrangements of the symbols 64 and 66. The term "different arrangements" as referred to here means that the arrangements do not overlap in a case in which each first pattern row 60 is read from any one direction. In a case in which the reading direction is changed for each of the plurality of first pattern rows 60, the arrangements may overlap. Hereinafter, a direction from the first marker M1 to the second marker M2 along the longitudinal direction (X-axis direction in Fig. 7) is defined as a forward direction, and conversely, a direction from the second marker M2 to the first marker M1 is defined as a reverse direction.

Specifically, in the first marker M1, the arrangement of the symbols 64 and 66 along the forward direction in at least one first pattern row 60 may be the same as the arrangement of the symbols 64 and 66 along the reverse direction in any one of the other first pattern rows 60. For example, in Fig. 7, in a case in which the first pattern row 60B is read in the forward direction, a circular symbol 64, a rectangular symbol 66, a circular symbol 64, and a rectangular symbol 66 are arranged in this order. In a case in which the first pattern row 60I is read in the reverse direction, a circular symbol 64, a rectangular symbol 66, a circular symbol 64, and a rectangular symbol 66 are arranged in this order. That is, the arrangement of the symbols 64 and 66 along the forward direction in the first pattern row 60B and the arrangement of the symbols 64 and 66 along the reverse direction in the first pattern row 60I are the same.

In this way, by allowing the overlap of the arrangement of the symbols in a case where one first pattern row 60 is read in the forward direction and another first pattern row 60 is read in the reverse direction in the first marker M1, the number of the symbols constituting the first pattern row 60 can be reduced. As a result, the first marker M can be made compact, thereby meeting the recent demand for miniaturization of the medical instrument. That is, even in a case in which the ultrasound probe 14 is small, the position and the orientation of the ultrasound probe 14 can be specified with high accuracy.

Similarly, in the second marker M2, the arrangement of the symbols 74 and 76 along the forward direction in at least one second pattern row 70 may be the same as the arrangement of the symbols 74 and 76 along the reverse direction in any one of the other second pattern rows 70. The effect in this case is also the same as that of the first marker M1.

In addition, as described above, the marker M can be configured to identify the first marker M1 and the second marker M2 from the surgical field image 21. In this case, since the forward direction and the reverse direction in the surgical field image 21 can also be specified from the relative positional relationship between the first marker M1 and the second marker M2, a predetermined (that is, correct) reading direction of the pattern row can be specified. Therefore, even in a case in which the arrangements of the symbols in a case in which one pattern row is read in the forward direction and another pattern row is read in the reverse direction overlap each other, the two pattern rows are not confused with each other, so that the specification accuracy can be ensured.

It is preferable that, in the first marker M1, the plurality of first pattern rows 60 are arranged such that only a combination of the first pattern rows 60 having a Hamming distance of 2 or more is included in a predetermined range in the circumferential direction. The Hamming distance referred to here is the number of locations where the symbols 64 and 66 at the same position are different in a combination of two first pattern rows 60. For example, in Fig. 7, the Hamming distance between the first pattern row 60A and the first pattern row 60B is 2.

Specifically, it is preferable that, for each first pattern row 60, the Hamming distance between itself and the other first pattern row 60 within a predetermined range (for example, the two in front and the two in back along the circumferential direction) is 2 or more. With the above-described configuration, the first pattern rows 60 having a close Hamming distance (that is, a Hamming distance of 1 or less) are less likely to be captured in the surgical field image 21 simultaneously. Therefore, it is possible to prevent erroneous detection in a case in which the first pattern row 60 is detected from the surgical field image 21, and it is possible to further improve the specification accuracy.

Similarly, it is preferable that, in the second marker M2, the plurality of second pattern rows 70 are arranged such that only a combination of the second pattern rows 70 having a Hamming distance of 2 or more is included in a predetermined range in the circumferential direction. The effect in this case is also the same as that of the first marker M1.

It is preferable that colors of the symbols 64 and 66 constituting the first pattern row 60 and colors of the symbols 74 and 76 constituting the second pattern row 70 are all the same. By making the first marker M1 and the second marker M2 the same color (monochrome), the manufacturing cost can be reduced. In addition, by not performing the identification of the symbols 64 and 66, and the identification of the symbols 74 and 76, by using color, it is possible to avoid being affected by the characteristics of camera 13B (for example, spectral sensitivity) and the characteristics of the illumination light (for example, color temperature and illuminance). Therefore, the position and the orientation of the ultrasound probe 14 can be specified with high accuracy in various situations.

Fig. 7 illustrates a form in which the number of the first pattern rows 60 included in the first marker M1 and the number of the second pattern rows 70 included in the second marker M2 are the same (13), but the present disclosure is not limited to this. For example, the number of the first pattern rows 60 included in the first marker M1 and the number of the second pattern rows 70 included in the second marker M2 may be different from each other.

In addition, Fig. 7 illustrates an example in which the number of the symbols 64 and 66 constituting the first pattern row 60 and the number of the symbols 74 and 76 constituting the second pattern row 70 are the same (4), but the present disclosure is not limited to this. For example, the number of the symbols 64 and 66 constituting the first pattern row 60 and the number of the symbols 74 and 76 constituting the second pattern row 70 may be different from each other.

It is preferable that the larger the number of the first pattern rows 60 included in the first marker M1 and the larger the number of the second pattern rows 70 included in the second marker M2, the more precisely the position and the orientation of the ultrasound probe 14 can be specified, but in practice, various constraints are applied. For example, the maximum number M of pattern rows that can be arranged is determined according to a shape of the ultrasound probe 14 and a resolution of the surgical field image 21.

For example, it is assumed that a diameter of the ultrasound probe 14 is designed to be 12 mm in order to be inserted into the trocar 17. In addition, it is assumed that it is known from an experiment that, in order to detect the marker M from the surgical field image 21 with sufficient accuracy while the ultrasound probe 14 takes various positions and orientations, it is necessary to set a size of the symbol to 1.2 mm square or more and to set an interval between the symbols in the longitudinal direction and the circumferential direction to 1.2 mm or more. In this case, the maximum number M of pattern rows that can be arranged in the circumferential direction of the ultrasound probe 14 is obtained by the following equation.

$$M = ((\text{diameter}) \times (\text{pi}))/((\text{size of symbol}) + (\text{interval between symbols}))$$
$$= (12 \times 3.14)/(1.2 + 1.2) = 15.7$$

In addition, in a case in which the maximum number M of pattern rows is obtained, the number of symbols constituting the pattern row can also be obtained. As described above, all the first pattern rows 60 in the first marker M1 are unique, and all the second pattern rows 70 in the second marker M2 are unique. That is, as the number of symbols constituting the pattern rows, the number that can express the maximum number M of unique pattern rows is required.

Specifically, in a case in which the number of types of symbols is a and the total number of symbols included in one pattern row is n, $a^n$ need only be equal to or more than the maximum number M of pattern rows. For example, in a case in which the number a of types of symbols is 2 and the maximum number M of pattern rows is 15, in order to satisfy $2^n \geq 15$, n is required to be 4 or more.

In addition, for example, the minimum number m of pattern rows arranged in the circumferential direction of the ultrasound probe 14 is preferably 7. This is because it is preferable that at least three pattern rows are captured in the surgical field image 21 from the viewpoint of redundancy in the circumferential direction. In the surgical field image 21, in a case in which at least three first pattern rows 60 can be detected from the first marker M1, the orientation of the ultrasound probe 14 in the circumferential direction can be specified even from the first marker M1 alone. Similarly, in the surgical field image 21, in a case in which at least three second pattern rows 70 can be detected from the second marker M2, the orientation of the ultrasound probe 14 in the circumferential direction can be specified even from the second marker M2 alone. In this case, in a case in which the number a of types of symbols is 2, in order to satisfy $2^n \geq 7$, n is required to be 3 or more.

In addition, the larger the number a of types of symbols and the total number n of symbols, the more the number of pattern rows that can be expressed increases, so that the Hamming distance between different pattern rows can be ensured, and erroneous detection can be prevented. On the other hand, the smaller the number a of types of symbols and the total number n of symbols, the simpler the detection processing, which can contribute to faster processing. In addition, the smaller the total number n of symbols, the more the space is saved, which is advantageous for miniaturizing the ultrasound probe 14. Therefore, the number a of types of symbols and the total number n of symbols may be appropriately set in consideration of the maximum number M and the minimum number m of pattern rows, the above circumstances, and the like.

The intermediate region 92 may be provided with information other than the marker M. The information other than the marker M may be, for example, position information indicating a position of the ultrasound transducer 14C, product information indicating identification information and specifications of the ultrasound probe 14, and direction information indicating a predetermined direction in the ultrasound probe 14. These types of information are expressed by, for example, symbols, figures, colors, and characters.

In Fig. 7, for example, the intermediate region 92 is provided with gradations 80. The gradations 80 are an example of the position information indicating the position of the ultrasound transducer 14C. The gradations 80 are arranged to correspond to the position of the ultrasound transducer 14C, that is, a distance from one end to the other end of the ultrasound transducer 14C in the longitudinal direction. In addition, the gradations 80 is further provided with information 80A indicating the center of the gradations 80 as an example of position information indicating a center position of the ultrasound transducer 14C.

In addition, in Fig. 7, as an example, the intermediate region 92 is provided with product information 82 indicating the identification information of the ultrasound probe 14 as a text string "FUJI123". In addition, the intermediate region 92 is provided with direction information 84 indicating the direction of the axis AX (longitudinal direction) of the distal end part 14B as a straight line.

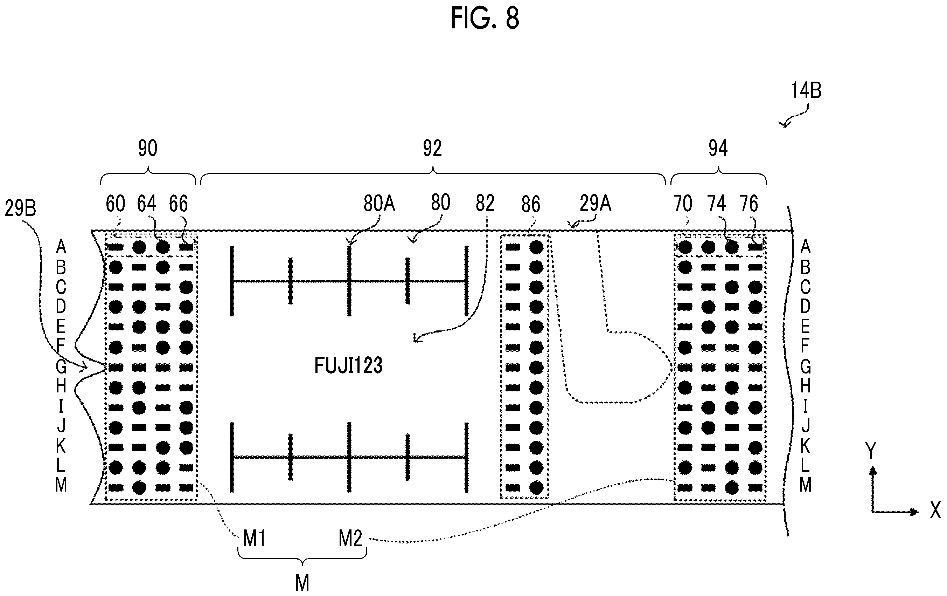

Fig. 8 shows a modification example of Fig. 7. In the example of Fig. 8, the intermediate region 92 is provided with direction information 86 indicating the reading directions of the first marker M1 and the second marker M2 as a figure. In the direction information 86 of Fig. 8, a direction from a rectangular figure to a circular figure indicates the reading directions of the first marker M1 and the second marker M2.

In this way, by utilizing the intermediate region 92 and providing various types of information related to the ultrasound probe 14, convenience can be improved. In particular, in a case in which the direction information 86 indicating the reading direction is printed, the accuracy of specifying the position and the orientation of the ultrasound probe 14 can be improved.

Further, when intermediate region 92 is provided with information in text format such as "FUJI123", it becomes easier for the user to judge whether camera 13B is or is not properly capturing marker M. Medical support device 11, as will be described later, performs processing for extracting marker M from the surgical field image 21. If, for example, marker M is outside the field of view of camera 13B, or the surgical field image 21 is blurred due to the lens being out-of-focus or contamination of the lens, appropriate extraction of marker M from the surgical field image 21 becomes difficult. Accordingly, the user must determine whether the surgical field image 21 is clear enough to allow extraction of marker M and, when it is not clear, take measures such as adjusting the field of view, adjusting focus, and cleaning the lens. In this case, because highly visible information such as "FUJI123" is provided in intermediate region 92, the user can more readily judge the clarity of the surgical field image 21. Therefore, the accuracy of specifying the position and the orientation of the ultrasound probe 14 can be improved.

Configuration of Medical Support Device 11

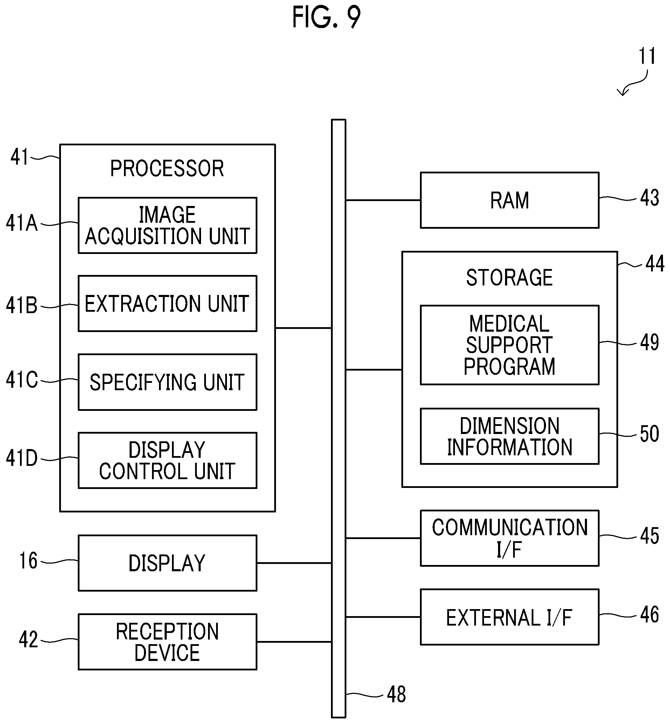

Next, a configuration of the medical support device 11 will be described. Fig. 9 shows an example of a hardware configuration of the medical support device 11. The medical support device 11 comprises a processor 41, a display 16, a reception device 42, a random access memory (RAM) 43, a storage 44, a communication interface (I/F) 45, and an external I/F 46. These units are connected to a bus 48 such as a system bus and a control bus, and can communicate with each other.

The medical support device 11 is operated by an operator such as the medical staff ST through the reception device 42. The reception device 42 includes a keyboard, a mouse, and the like (not shown), and receives an instruction from the operator. The reception device 42 may be a device that receives touch input, such as a touch panel, a device that receives voice input, such as a microphone, a device that receives gesture input, such as a camera, or the like.

Examples of the display 16 include a liquid crystal display and an electro-luminescence (EL) display. As described above, there are two displays 16, and various types of information are displayed on each display 16, in addition to the surgical field image 21 and the ultrasound image 22.

The processor 41 is, for example, a central processing unit (CPU), and integrally controls the respective units of the medical support device 11 in accordance with a control program and executes various types of processing in accordance with various types of application programs.

The storage 44 is a non-volatile storage device that stores various programs, various parameters, and the like. Examples of the storage 44 include a hard disk drive (HDD) and a solid state drive (SSD). In addition, the storage 44 stores a medical support program 49 for causing a computer to function as the medical support device 11.

The RAM 43 is a memory that transitorily stores information, and is used as a work memory by the processor 41. Examples of the RAM 43 include a dynamic random access memory (DRAM) or a static random access memory (SRAM).

The communication I/F 45 is connected to a network (not shown) such as a local area network (LAN) and/or a wide area network (WAN), and performs transmission control in accordance with a communication protocol defined in various types of wired or wireless communication standards.

The external I/F 46 is, for example, a universal serial bus (USB) interface, and is used for connection to peripheral devices such as a printer and a memory card.

The processor 41 executes medical support processing by reading out the medical support program 49 from the storage 44 and executing the medical support program 49 on the RAM 43. The medical support processing is executed by the processor 41 operating as an image acquisition unit 41A, an extraction unit 41B, a specifying unit 41C, and a display control unit 41D.

In addition, the storage 44 stores dimension information 50. The dimension information 50 includes information indicating dimensions of the ultrasound probe 14, specifically, a positional relationship of the marker M in the distal end part 14B of the ultrasound probe 14 and a positional relationship of the guide groove 29 in the distal end part 14B.

The positional relationship of the marker M in the distal end part 14B is, for example, information on the position and the orientation at which the arrangement of the pattern rows included in the marker M is provided with respect to the axial direction and the circumferential direction of the distal end part 14B. The positional relationship of the guide groove 29 in the distal end part 14B is a linear distance in the axial direction along the axis AX of the distal end part 14B between a reference point of the distal end part 14B and the guide groove 29, an inclination angle θ (including θ1 and θ2) of the guide groove 29 with respect to the axial direction of the distal end part 14B, or the like.

The image acquisition unit 41A acquires the surgical field image 21 and the ultrasound image 22. For example, the image acquisition unit 41A acquires the surgical field image 21 from a device including a processor of the endoscope 13 via the external I/F 46 or the communication I/F 45. In addition, for example, the image acquisition unit 41A acquires the ultrasound image 22 from the device including the processor of the ultrasound probe 14 via the external I/F 46 or the communication I/F 45. The medical support device 11 may have the processor of the endoscope 13 and/or the processor of the ultrasound probe 14. The surgical field image 21 is an example of an optical image obtained by optically imaging the ultrasound probe 14 inserted into the body with the camera 13B.

The extraction unit 41B extracts at least a part of pattern rows from among the plurality of first pattern rows 60 and the plurality of second pattern rows 70 from the surgical field image 21. Specifically, it is preferable that the extraction unit 41B extracts at least three pattern rows in total from among the plurality of first pattern rows 60 and the plurality of second pattern rows 70. By extracting three or more pattern rows, the specifying unit 41C can satisfactorily specify the position and the orientation of the ultrasound probe 14 in the longitudinal direction and the circumferential direction.

For example, the extraction unit 41B may extract the pattern row using an image processing method such as pattern matching. The processor 41 may extract the pattern row by searching for the morphological features of the markers M, such as the symbols 64, 66, 74, and 76, from the surgical field image 21.

In addition, for example, the pattern row may be extracted using an artificial intelligence (AI) technology using a machine learning model instead of a rule-based method such as pattern matching. As such a machine learning model, for example, a neural network model such as a convolutional neural network (CNN) that has been trained in advance by using the surgical field image 21 as an input and a pattern row in the input surgical field image 21 as an output can be applied.

Specifically, the machine learning model may output, as a heat map, the probability that each pixel of the surgical field image 21 is the marker M. In this case, by specifying a region in which the probability is equal to or higher than a predetermined threshold value as one of the symbols 64, 66, 74, and 76, a type of the symbol 64, 66, 74, or 76 can be determined from a shape of the region. In addition, coordinates of the center or the centroid of the region can be specified as coordinates of the symbol 64, 66, 74, or 76. From the positional relationship between the plurality of symbols 64, 66, 74, and 76 obtained in this way, the position and the orientation (for example, the extension direction) of the pattern row can be specified.

As described below, the specifying unit 41C specifies the position and the orientation of the ultrasound probe 14, more specifically, the position and the orientation of the distal end part 14B in the surgical field SF based on at least the part of the pattern rows extracted from the surgical field image 21.

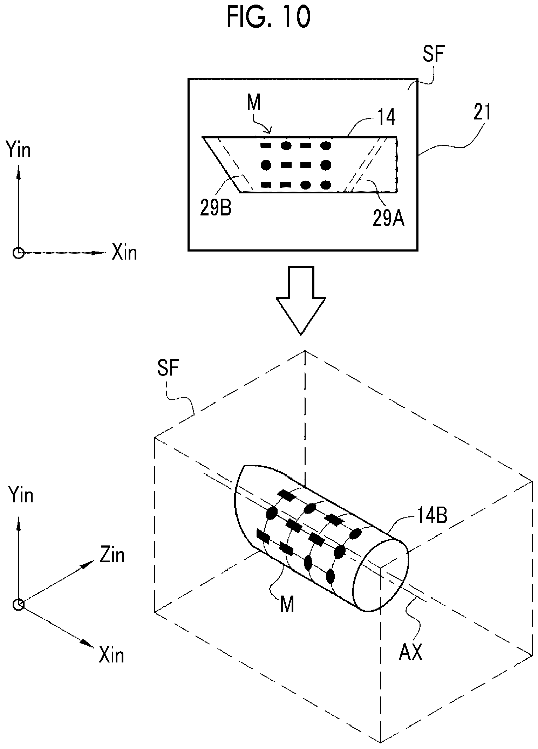

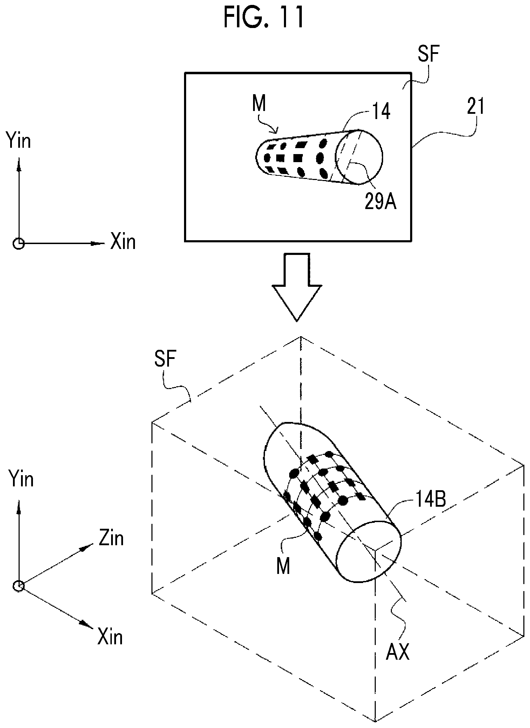

Figs. 10 and 11 conceptually show a correspondence relationship between the position and the orientation of the distal end part 14B of the ultrasound probe 14 having the marker M in the surgical field image 21, and the position and the orientation of the distal end part 14B in the surgical field SF defined as a three-dimensional space. A Zin axis of the surgical field SF, which is the three-dimensional space, is a direction parallel to an imaging optical axis of the camera 13B, an Xin-Yin plane (hereinafter, simply referred to as an XY plane) is a plane parallel to an imaging surface of the camera 13B and orthogonal to the imaging optical axis. In Figs. 10 and 11, the XY plane is parallel to a screen of the surgical field image 21. The surgical field image 21 is a projection image obtained by projecting the surgical field SF from one viewpoint. In Figs. 10 and 11, a grid in which each symbol corresponds to a lattice point is displayed so that the positional relationship between the symbols included in each pattern row is easy to understand.

Fig. 10 shows a state in which the axial direction of the distal end part 14B of the ultrasound probe 14 is orthogonal to the imaging optical axis of the camera 13B in the surgical field SF that is the three-dimensional space (more specifically, a state in which the axis AX of the distal end part 14B is parallel to the Xin axis). In a case in which the distal end part 14B is in such an orientation, the surgical field image 21 is in a state in which the longitudinal direction (that is, the arrangement direction of the symbols in each pattern row) is parallel to the Xin axis and the circumferential direction (that is, the arrangement direction of the plurality of pattern rows) is parallel to the Yin axis. Therefore, the symbols are reflected in the surgical field image 21 at equal intervals.

On the other hand, Fig. 11 shows a state in which the axial direction of the distal end part 14B of the ultrasound probe 14 is not orthogonal to the imaging optical axis of the camera 13B in the surgical field SF that is the three-dimensional space and is inclined in a depth direction parallel to the imaging optical axis. The orientation shown in Fig. 11 is a state in which the axis AX of the distal end part 14B is rotated by approximately -15° about the Yin axis from the orientation shown in Fig. 10. In a case in which the distal end part 14B is in such an orientation, in the surgical field image 21, the farther away from the camera 13B in the depth direction, the shorter the interval between the pattern rows, and the shorter the interval between the symbols.

In this way, the form of the marker M reflected in the surgical field image 21 changes depending on the orientation of the distal end part 14B. The processor 41 specifies the orientation of the distal end part 14B of the ultrasound probe 14 in the surgical field SF based on the form of the marker M in the surgical field image 21. Specifically, the direction of the axis AX of the distal end part 14B in the surgical field SF, that is, the axial direction of the distal end part 14B is detected as the orientation of the distal end part 14B. For example, the extension direction of one pattern row extracted by the extraction unit 41B matches the direction of the axis AX.

In addition, in a case in which the position of the distal end part 14B is changed in the surgical field SF, the position of the marker M reflected in the surgical field image 21 is also changed. The processor 41 specifies the position of the distal end part 14B in the surgical field SF based on the position of the marker M. The position of the distal end part 14B is, for example, a position of the reference point of the distal end part 14B, such as a distal end position of the distal end part 14B. Further, an imaging distance from the camera 13B to the marker M in the surgical field SF (that is, a distance in the Zin axis direction parallel to the imaging optical axis) can be calculated based on a focal length of the camera 13B and the size of the marker M reflected in the surgical field image 21. The processor 41 derives position coordinates of the reference point of the distal end part 14B in the surgical field SF based on the dimension information 50 of the distal end part 14B, which includes the imaging distance and the known dimensions of the marker M.

The specifying unit 41C specifies the position and the orientation of the guide groove 29 of the ultrasound probe 14 in the surgical field SF based on the specified position and orientation of the distal end part 14B and the positional relationship of the guide groove 29 in the distal end part 14B included in the dimension information 50 of the ultrasound probe 14.

In the example of Fig. 10, the position of the distal end part 14B of the ultrasound probe 14 is specified as information such as the position coordinates $(X01, Y01, Z01)$ of the reference point of the distal end part 14B in the surgical field SF. The orientation of the distal end part 14B is defined as the axial direction of the distal end part 14B in the surgical field SF. For example, the axis AX of the distal end part 14B is specified as information indicating that it is parallel to the XY plane and the XZ plane and that is orthogonal to the YZ plane.

In addition, the position of the first guide groove 29A is specified as information such as the position coordinates $(X11, Y11, Z11)$ of the reference point in the surgical field SF. The position of the second guide groove 29B is specified as information such as the position coordinates $(X21, Y21, Z21)$ of the reference point in the surgical field SF. The orientation of each of the first guide groove 29A and the second guide groove 29B is defined as the orientation with respect to the axis AX, and serves as the known inclination angle $\theta1$ and inclination angle $\theta2$ as the dimension information 50.

In the example of Fig. 11, the position of the distal end part 14B of the ultrasound probe 14 is specified as information such as the position coordinates $(X02, Y02, Z02)$ of the reference point of the distal end part 14B in the surgical field SF. The orientation of the distal end part 14B is defined as the axial direction of the distal end part 14B in the surgical field SF. For example, the axis AX of the distal end part 14B is specified as information indicating that it is -15° with respect to the XY plane, parallel to the XZ plane, and is 75° with respect to the YZ plane.

In addition, the position of the first guide groove 29A is specified as information such as the position coordinates $(X12, Y12, Z12)$ of the reference point in the surgical field SF. The position of the second guide groove 29B is specified as information such as the position coordinates $(X22, Y22, Z22)$ of the reference point in the surgical field SF. The orientation of each of the first guide groove 29A and the second guide groove 29B is defined as the orientation with respect to the axis AX, and serves as the known inclination angle $\theta1$ and inclination angle $\theta2$ as the dimension information 50.

The above-described specification method can also be applied to a case in which the position and the orientation of the ultrasound probe 14 are specified in a case in which only one of the first marker M1 or the second marker M2 is reflected in the surgical field image 21. On the other hand, in a case in which both the first marker M1 and the second marker M2 are reflected in the surgical field image 21, the position and the orientation of the ultrasound probe 14 can be specified by using the positional relationship between the first marker M1 and the second marker M2.

For example, the specifying unit 41C may specify the positional relationship between the first marker M1 and the second marker M2 based on the shape of the ultrasound probe 14 included in the surgical field image 21. For example, the shape of the ultrasound probe 14, such as the first guide groove 29A and the second guide groove 29B, and the positional relationship between the first marker M1 and the second marker M2 are known. Therefore, in a case in which the shape of the ultrasound probe 14 can be detected from the surgical field image 21, the first marker M1 and the second marker M2 can be identified. In a case in which the shape of the ultrasound probe 14 is detected from the surgical field image 21, for example, an image processing method such as pattern matching can be used.

In addition, for example, an AI technology using a machine learning model may be used instead of a rule-based method such as pattern matching. As such a machine learning model, for example, a trained model such as CNN, which has been trained in advance by using the surgical field image 21 as an input and the first region 90 and the second region 94 in the input surgical field image 21 as an output, can be applied. That is, the trained model is a model that classifies the ultrasound probe 14 in the surgical field image 21 into the first region 90, the second region 94, and the other region.

The specifying unit 41C may detect the first region 90 and the second region 94 from the surgical field image 21 using the trained model, and specify the positional relationship between the first marker M1 and the second marker M2 based on the detected first region 90 and second region 94. Specifically, it is assumed that both the first marker M1 and the second marker M2 are located on a straight line connecting the first region 90 and the second region 94 detected by the trained model. Therefore, the specifying unit 41C may specify the first marker M1 and the second marker M2 that are configured by a pattern arrangement such that their directions with respect to the straight line are aligned.

The specifying unit 41C specifies the position and the orientation of the ultrasound probe 14 based on at least the part of the pattern rows that is extracted and the specified positional relationship between the first marker M1 and the second marker M2. As a result, in addition to the pattern row, the position and the orientation of the entire ultrasound probe 14 can be specified from the positional relationship between the first marker M1 and the second marker M2 reflected in the surgical field image 21, thereby further improving the specification accuracy.

In addition, the specifying unit 41C derives the first puncture line NR1 of the biopsy needle 18 in the surgical field image 21 based on the specified position and orientation of the guide groove 29. Specifically, the specifying unit 41C derives the first puncture line NR1 that serves as a straight line along the inclination angle $\theta1$ of the first guide groove 29A in the surgical field SF based on the position coordinates of the first guide groove 29A in the surgical field SF defined as the three-dimensional space and the inclination angle $\theta1$ of the distal end part 14B with respect to the axis AX.

For example, the specifying unit 41C sets the position of the first guide groove 29A as a base point, and sets a line segment that is inclined from the position of the first guide groove 29A at the inclination angle $\theta1$ of the first guide groove 29A as the first puncture line NR1. Then, the specifying unit 41C derives the first puncture line NR1 in the surgical field image 21 by converting the first puncture line NR1 into a two-dimensional coordinate system of the surgical field image 21. The same applies to the second guide groove 29B.

The display control unit 41D executes control of displaying the surgical field image 21 and the ultrasound image 22 on the display 16. In addition, the display control unit 41D performs control such that the derived first puncture line NR1 is superimposed and displayed on the surgical field image 21. Specifically, the term "superimpose and display" refers to control of generating a composite image in which the first puncture line NR1 is superimposed on the surgical field image 21 and displaying the generated composite image on the display 16. As a result, the surgical field image 21 on which the first puncture line NR1 is superimposed and displayed is displayed on the display 16.

Since the surgical field image 21 is output to the display 16 as a video, the position and the orientation of the first puncture line NR1 in the surgical field image 21 displayed on the display 16 are also updated to reflect the positions of the endoscope 13, the ultrasound probe 14, and the like in a real space imaged by the camera 13B.

Actions and effects of the technology of the present disclosure will be described using, as an example, a case in which the liver LV is punctured by the biopsy needle 18 with the tumor 27 in the liver LV, which is an organ as a puncture target, as a target position. As shown in Figs. 1 and 5, for example, the medical staff ST adjusts the position and the orientation of the ultrasound transducer 14C provided at the distal end part 14B of the ultrasound probe 14 while observing the ultrasound image 22 on which the second puncture line NR2 is superimposed and displayed. Specifically, the position and the orientation of the ultrasound transducer 14C are adjusted to a position at which the second puncture line NR2 serving as the extension line of the guide groove 29 passes through the tumor 27.

The position and the orientation of the first puncture line NR1 superimposed and displayed on the surgical field image 21 are changed in response to the change in the position and the orientation of the ultrasound probe 14. In a case in which the second puncture line NR2 is displayed to pass through the tumor 27 in the ultrasound image 22, the second puncture line NR2 indicates an ideal puncture path of the biopsy needle 18 in the liver LV. In this state, the position and the orientation of the ultrasound probe 14 are fixed while the puncture is being performed.

Then, the first puncture line NR1 in the surgical field image 21 in this state indicates, for example, an ideal puncture path of the biopsy needle 18 in the body cavity from the body surface BS of the patient PT to the surface of the liver LV as the puncture target. The medical staff ST adjusts the position and the orientation of the biopsy needle 18 such that the biopsy needle 18 is inserted along the ideal first puncture line NR1 outside the body while observing the surgical field image 21 on which the ideal first puncture line NR1 is superimposed and displayed.

Specifically, an approximate insertion position NP corresponding to the first puncture line NR1 is searched for, and the body surface BS of the patient PT is punctured by the biopsy needle 18 from the insertion position NP. Then, the biopsy needle 18 is inserted into the body cavity toward the first guide groove 29A of the ultrasound probe 14 while adjusting the position and the orientation of the biopsy needle 18 while viewing the display of the first puncture line NR1. Since the first puncture line NR1 is displayed with the first guide groove 29A as a base point, the first guide groove 29A can be easily reached by inserting the biopsy needle 18 along the first puncture line NR1.

After the biopsy needle 18 reaches the first guide groove 29A, the tumor 27 as the target position can be punctured by the biopsy needle 18 by puncturing the liver LV with the biopsy needle 18 along the second puncture line NR2 while checking the second puncture line NR2 of the ultrasound image 22 as shown in Figs. 1, 2, and 5. As an example, the tumor 27 is cauterized in a state in which the tumor 27 is punctured by the biopsy needle 18.

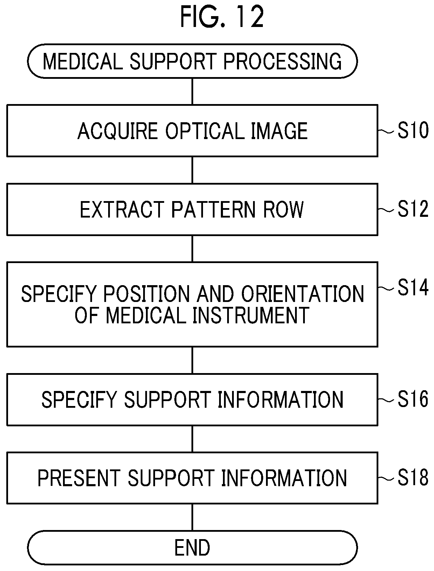

Next, an action of the medical support device 11 will be described with reference to Fig. 12. In the medical support device 11, the medical support processing shown in Fig. 12 is executed by the processor 41 executing the medical support program 49. This processing is executed, for example, in a case in which a user gives an instruction to start execution via the reception device 42.

In step S10, the image acquisition unit 41A acquires an optical image (for example, the surgical field image 21) by optically imaging a medical instrument (for example, the ultrasound probe 14) inserted into the body with the camera 13B. In step S12, the extraction unit 41B extracts at least a part of pattern rows from among the plurality of first pattern rows 60 and the plurality of second pattern rows 70 from the optical image acquired in step S10.

In step S14, the specifying unit 41C specifies a position and an orientation of the medical instrument based on at least the part of the pattern rows extracted in step S12. In step S16, the specifying unit 41C specifies, as support information, a puncture path of the biopsy needle 18 in the optical image acquired in step S10 based on the position and the orientation of the medical instrument (for example, the guide groove 29) specified in step S14. In step S18, the display control unit 41D performs control to present the support information specified in step S16 on the display 16, and ends this processing.

As described above, the medical instrument (ultrasound probe 14) according to the present embodiment includes the first region 90 provided with the first marker M1, the intermediate region 92 provided with no marker M, and the second region 94 provided with the second marker M2, along the longitudinal direction. The first marker M1 includes the plural first pattern rows 60 arranged along the direction that intersects with the longitudinal direction. Each of the first pattern rows 60 is configured by the plural symbols 64 and 66 arranged along the longitudinal direction, and the arrangement of the symbols 64 and 66 in each first pattern row 60 differs from the arrangement of the symbols 64 and 66 in any first pattern row 60 adjacent thereto. The second marker M2 includes the plural second pattern rows 70 arranged along the direction that intersects with the longitudinal direction. Each of the second pattern rows 70 is configured by the plural symbols 74 and 76 arranged along the longitudinal direction, and the arrangement of the symbols 74 and 76 in each second pattern row 70 differs from the arrangement of the symbols 74 and 76 in any second pattern row 70 adjacent thereto.

That is, with the medical instrument according to the present embodiment, redundancy is ensured in both the longitudinal direction and the circumferential direction in the marker M for specifying the posture and the orientation of the medical instrument. Therefore, it is possible to specify the position and the orientation of the medical instrument with high accuracy in various situations, and to improve the practicality. In a case in which both the first marker M1 and the second marker M2 can be detected, the position and the orientation of the entire medical instrument can be specified from the positional relationship between the first marker M1 and the second marker M2, thereby further improving the specification accuracy.

In addition, in the technology of the present disclosure, the hardware configuration for specifying the position and the orientation of the medical instrument such as the ultrasound probe 14 can be achieved by simply providing the marker M at the insertion part of the medical instrument. Therefore, a large-scale device such as a magnetic system is not required. In addition, even in a case in which the medical instrument in the related art is diverted, the configuration is simpler than a configuration in which a light source for the marker M is incorporated because it is necessary to modify only the exterior of the insertion part. Therefore, the medical support device 11 can display the support information (for example, the first puncture line NR1 of the biopsy needle 18 at an appropriate position) according to the position or the orientation of the medical instrument with a simple configuration.

In addition, in the above-described embodiment, the medical instrument to be inserted into the body of the patient PT is the ultrasound probe 14 (an example of a medical probe) that can observe the internal structure of the organ. The treatment of puncturing the organ inside the body with the biopsy needle 18 is often performed using the medical probe capable of observing the internal structure of the organ. Therefore, as in the above-described embodiment, the technology of the present disclosure is particularly effective in a case in which the medical probe is used as the medical instrument.

Further, the ultrasound probe 14 is used in combination with the biopsy needle 18 relatively frequently. Therefore, the technology of the present disclosure is more effective in a case in which the ultrasound probe 14 is used as the medical probe. As the medical probe capable of observing the internal structure of the organ, a probe other than the ultrasound probe 14 may be used, such as an optical coherence tomography (OCT) probe.

As the medical instrument, a medical instrument other than the medical probe capable of observing the internal structure of the organ can also be applied. For example, as the medical instrument, a treatment tool may be used, which does not have an internal structure observation function and has only the guide groove 29 of the biopsy needle 18 at the distal end part. For example, in a case in which the tumor exists on the surface of the organ, in a case in which the treatment of puncturing the tumor on the surface with the biopsy needle 18 is performed, even with the treatment tool that does not have the internal structure observation function, the biopsy needle 18 can be appropriately guided as long as the guide groove 29 is provided. In this case, for example, the medical staff ST positions the guide groove 29 of the treatment tool at a position of the surface of the organ corresponding to the tumor, and in this state, the medical staff ST punctures the tumor with the biopsy needle 18 through the guide groove 29.

Further, as the medical instrument, a treatment tool such as a simple rod without including the guide groove 29 may be used. In a case in which the tumor exists on the surface of the organ, it is possible to point to the tumor even with such a treatment tool. Even in a case in which the first puncture line NR1 is simply superimposed and displayed on the surgical field image 21 in which the treatment tool points to the tumor on the surface of the organ, the first puncture line NR1 serves as a guide for checking a puncture direction of the biopsy needle 18 or the like. Therefore, the technology of the present disclosure is effective even for a medical instrument that is a treatment tool without including the guide groove 29.

In addition, in the above-described embodiment, the shapes of the symbols 64, 66, 74, and 76 are described as being circular, rectangular, triangular, and the like, but the present disclosure is not limited to this. Figs. 13 to 18 show other examples of the shapes of the symbols 64, 66, 74, and 76. The symbols 64 and 74 shown in Fig. 13 have a shape (that is, a ring shape) in which a circle is circularly cut out or a shape in which circles having different sizes and colors overlap each other. The symbols 66 and 76 shown in Fig. 14 have a shape in which a rectangle is circularly cut out or a shape in which a circle of a different color is superimposed on a rectangle.

The symbols 64 and 74 shown in Fig. 15 have a shape in which a circle is circularly cut out and a cross mark is disposed in the cut-out portion. The symbols 66 and 76 shown in Fig. 16 have a shape in which a rectangle is circularly cut out and a cross mark is disposed in the cut-out portion. As described above, the symbols 64, 66, 74, and 76 may be represented by a combination of a plurality of figures. In addition, for example, the symbol may be represented by a figure such as a polygonal shape, a star shape, and various marks, or may be represented by characters.

In addition, in the above-described embodiment, the form in which the distal end part 14B of the insertion part 14A of the ultrasound probe 14 is provided with the marker M has been described, but the present invention is not limited to this. The marker M need only be provided to a portion of various medical instruments that is inserted into the body of the patient PT, and may be provided, for example, to a middle portion of the insertion part 14A and a base end side of the insertion part 14A.

In addition, in the above-described embodiment, the cauterization has been described as an example of the function of the biopsy needle 18, but the function of the biopsy needle 18 is not limited to this. In addition, although the biopsy needle 18 has been described as an example of the treatment tool, a treatment tool for injecting a fluorescent agent such as indocyanine green (ICG), a biopsy needle used for tissue collection for performing a biopsy, forceps, and the like may be applied instead of the biopsy needle 18.

In addition, in the above-described embodiment, the body cavity such as the abdominal cavity and the thoracic cavity has been described as the inside of the body, but the inside of the body may be an upper digestive tract such as an esophagus, a lower digestive tract such as an intestine, and a tubular organ such as a bronchus. In a case in which the technology of the present disclosure is applied to a surgical field in the tubular organ, for example, the marker M is provided in a base end part of a soft endoscope to be inserted into the tubular organ.

In addition, in the above-described embodiment, an example in which the puncture path (first puncture line NR1 and second puncture line NR2) of the biopsy needle 18 is used as the support information according to the position or the orientation of the medical instrument has been described, but the present disclosure is not limited to this. For example, the medical support device 11 may provide the support information according to the position or the orientation of the ultrasound probe 14 by superimposing and displaying the ultrasound image 22 according to the position or the orientation of the ultrasound probe 14 on the surgical field image 21, in addition to or instead of the puncture path of the biopsy needle 18.

Here, an imaging range of the ultrasound image 22 is determined according to the position and the orientation of the ultrasound transducer 14C, and more specifically, is a fan-shaped range with the ultrasound transducer 14C as a base point. The disposition position of the ultrasound transducer 14C in the ultrasound probe 14 is known and is included in the dimension information 50 of the ultrasound probe 14. Therefore, in a case in which the position and the orientation of the ultrasound probe 14 are known, the position and the orientation of the ultrasound transducer 14C can also be specified, so that the imaging range of the ultrasound image 22 in the surgical field image 21 can be specified.

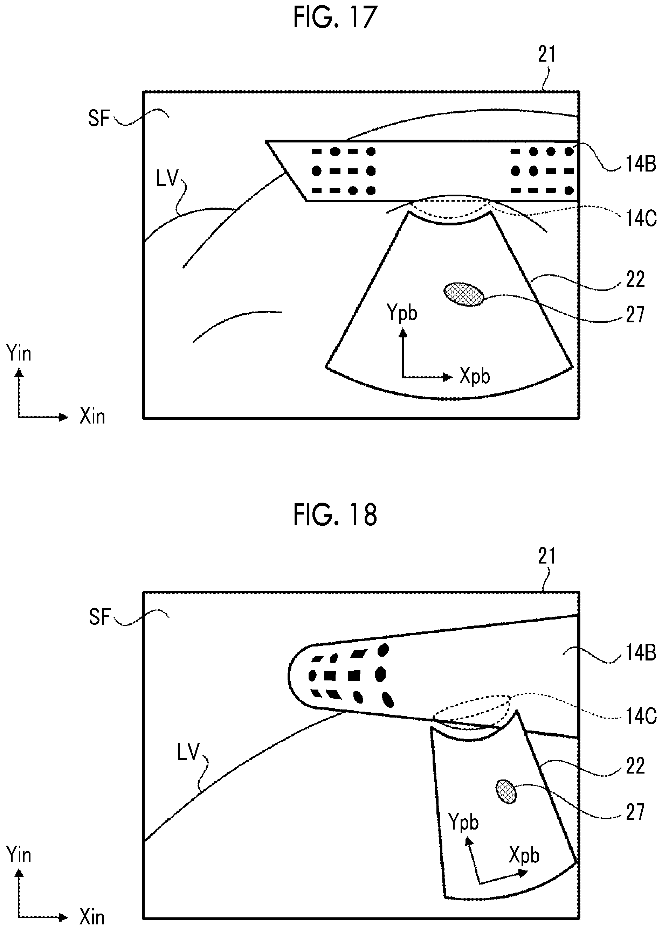

Figs. 17 and 18 show an example in which the ultrasound image 22 corresponding to the position and the orientation of the ultrasound probe 14 is superimposed and displayed on the surgical field image 21. Fig. 17 shows a state in which the axial direction of the distal end part 14B of the ultrasound probe 14 is orthogonal to the imaging optical axis of the camera 13B, similarly to Fig. 10. In this state, the Xin-Yin plane in which the surgical field image 21 is formed in the surgical field SF and the Xpb-Ypb plane in which the ultrasound image 22 is formed in the surgical field SF are parallel to each other.

On the other hand, Fig. 18 shows a state in which the axial direction of the distal end part 14B of the ultrasound probe 14 is not orthogonal to the imaging optical axis of the camera 13B and is inclined in the depth direction parallel to the imaging optical axis, similarly to Fig. 11. In this state, the Xin-Yin plane in which the surgical field image 21 is formed in the surgical field SF and the Xpb-Ypb plane in which the ultrasound image 22 is formed in the surgical field SF are not parallel to each other. The medical support device 11 may perform deformation such as projective transformation, affine transformation, movement, rotation, enlargement, and reduction on the ultrasound image 22 in consideration of a difference in coordinate systems between the surgical field image 21 and the ultrasound image 22, and then superimpose and display the ultrasound image 22 on the surgical field image 21.

Specifically, as described above, the specifying unit 41C specifies the position and the orientation of the distal end part 14B of the ultrasound probe 14 in the surgical field image 21 using the marker M. In addition, the specifying unit 41C specifies the position and the orientation of the ultrasound transducer 14C in the surgical field SF based on the specified position and orientation of the distal end part 14B and the positional relationship of the ultrasound transducer 14C included in the dimension information 50. Then, the specifying unit 41C performs deformation processing such as projective transformation such that the imaging range (Xpb-Ypb plane) of the ultrasound image 22 is formed in the surgical field image 21 (Xin-Yin plane), based on the specified position and orientation of the ultrasound transducer 14C. The display control unit 41D performs control such that the deformed ultrasound image 22 is superimposed and displayed on the surgical field image 21.

With such support information, for example, even in a case in which it is difficult to visually recognize the position and the orientation of the ultrasound transducer 14C in the surgical field image 21 due to shield by an organ or the like and a positional relationship with the camera 13B, the position and the orientation can be easily grasped. Therefore, it is easy to adjust the position and the orientation of the ultrasound probe 14 so that a desired region (for example, the tumor 27) is reflected in the ultrasound image 22, and it is possible to contribute to the improvement of the operability of the ultrasound probe 14.

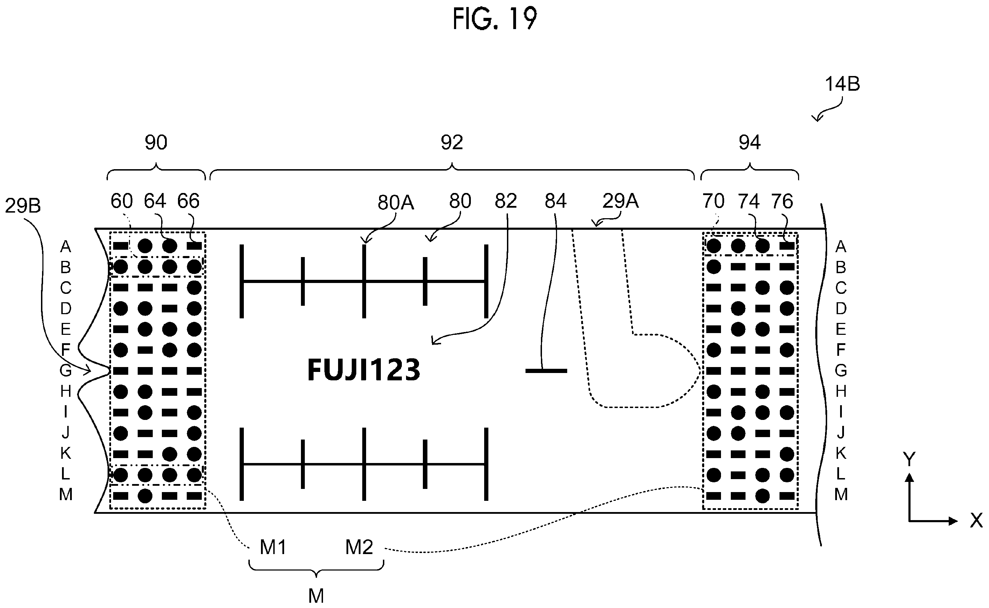

In addition, in the above-described embodiment, as shown in Fig. 7, the form in which the plural first pattern rows 60 have mutually different arrangements of the symbols 64 and 66 has been described, but the present invention is not limited to this. In the medical instrument of the present embodiment, it suffices that the arrangement of the symbols 64 and 66 in each first pattern row 60 differs from the arrangement of the symbols 64 and 66 in any first pattern row 60 adjacent thereto. In other words, within the plural first pattern rows 60, there may be a combination having identical symbol arrangements of symbols 64 and 66. Fig. 19 shows, as a modification of Fig. 7, an example configuration that includes such a combination having identical symbol arrangements of symbols 64 and 66. In Fig. 19, first pattern row 60B and first pattern row 60L have identical symbol arrangements, each being composed of four symbols 64.

Similarly, it suffices that the arrangement of the symbols 74 and 76 in each second pattern row 70 differs from the arrangement of the symbols 74 and 76 in any second pattern row 70 adjacent thereto. In other words, within the plural second pattern rows 70, there may be a combination having identical symbol arrangements of symbols 74 and 76.

When a pattern row identical to another pattern row is extracted, the specifying unit 41C may distinguish the pattern rows having identical arrangements by using other pattern rows within a predetermined range of the pattern row in question. For example, suppose a pattern row (hereinafter, "overlapping pattern row") is extracted whose arrangement is identical and for which it is unclear whether it is first pattern row 60B or first pattern row 60L. In this case, when first pattern rows 60A and/or 60C, which are adjacent in the circumferential direction to the overlapping pattern row, are also extracted, the specifying unit 41C may determine that the overlapping pattern row is first pattern row 60B (that is, not first pattern row 60L). Further, for example, when at least one of second pattern rows 70A to 70C, which are second pattern rows positioned close to the overlapping pattern row in the circumferential direction, is extracted, the specifying unit 41C may likewise determine that the overlapping pattern row is first pattern row 60B.

For example, when the overlapping pattern rows, such as first pattern rows 60B and 60L, are provided on the right and left side surfaces of ultrasound probe 14, the specifying unit 41C may determine whether the portion of ultrasound probe 14 captured in the surgical field image 21 corresponds to the right side surface or the left side surface. Specifically, the specifying unit 41C may identify which side surface appears in the surgical field image 21 on the basis of at least one of the shape of ultrasound probe 14 visible in the surgical field image 21 and the product information 82 (e.g., the text "FUJI123") provided in intermediate region 92. For example, the side surface on which the first pattern rows 60A to 60F are provided is regarded as the right side surface, and the side surface on which the first pattern rows 60H to 60M are provided is regarded as the left side surface. In this case, if the specifying unit 41C determines that the right side surface is captured, the overlapping pattern row appearing in the surgical field image 21 can be specified as the first pattern row 60B.

In addition, in the above-described embodiment, for example, the following various processors can be used as a hardware structure of processing units that execute various types of processing, such as the image acquisition unit 41A, the extraction unit 41B, the specifying unit 41C, and the display control unit 41D. As described above, the various processors include a programmable logic device (PLD) as a processor of which the circuit configuration can be changed after manufacture, such as a field programmable gate array (FPGA), a dedicated electrical circuit as a processor having a dedicated circuit configuration for executing specific processing such as an application specific integrated circuit (ASIC), and the like, in addition to the CPU as a general-purpose processor that functions as various processing units by executing software (programs).

One processing unit may be configured of one of the various processors, or may be configured of a combination of the same or different kinds of two or more processors (for example, a combination of a plurality of FPGAs or a combination of the CPU and the FPGA). In addition, a plurality of processing units may be configured of one processor

As an example in which a plurality of processing units are configured of one processor, first, as typified by a computer such as a client or a server, there is an aspect in which one processor is configured of a combination of one or more CPUs and software, and this processor functions as a plurality of processing units. Second, as typified by a system on chip (SoC) or the like, a processor that realizes the functions of the entire system including the plurality of processing units by using one integrated circuit (IC) chip is used. As described above, various processing units are configured by using one or more of the various processors as a hardware structure.

Furthermore, as the hardware structure of these various processors, more specifically, an electric circuit (circuitry) in which circuit elements such as semiconductor elements are combined can be used.

In addition, in the above-described embodiment, the medical support program 49 has been described as being stored (installed) in the storage 44 in advance, but the present disclosure is not limited to this. The program may be provided in a form of being recorded on a recording medium, such as a compact disc read only memory (CD-ROM), a digital versatile disc read only memory (DVD-ROM), or a universal serial bus (USB) memory. In addition, the program may be downloaded from an external device via a network. Further, the technology of the present disclosure extends to a storage medium that non-transitorily stores a program in addition to the program.

In the technology of the present disclosure, the embodiment and the modification examples described above can be combined as appropriate. The content of the above description and the content of the drawings are detailed explanations of the parts relating to the technology of the present disclosure, and are merely examples of the technology of the present disclosure. For example, the above description related to the configuration, the function, the operation, and the effect is the description related to the examples of the configuration, the function, the operation, and the effect of the parts according to the technology of the present disclosure. Therefore, unnecessary portions may be deleted or new elements may be added or replaced in the contents described and illustrated above, without departing from the scope and spirit of the technology of the present disclosure.

The following appendices are disclosed with regard to the above embodiment.

Appendix 1

A medical instrument comprising a first region provided with a first marker, an intermediate region not provided with a marker, and a second region provided with a second marker, which are disposed in a longitudinal direction, in which:

the first marker includes plural first pattern rows arranged along a direction that intersects with the longitudinal direction, each of the first pattern rows being configured by plural symbols arranged along the longitudinal direction, an arrangement of the symbols in each first pattern row being different from an arrangement of the symbols in any first pattern row adjacent thereto, and

the second marker includes plural second pattern rows arranged along the direction that intersects with the longitudinal direction, each of the second pattern rows being configured by plural symbols arranged along the longitudinal direction, an arrangement of the symbols in each second pattern row being different from an arrangement of the symbols in any second pattern row adjacent thereto.

Appendix 2

The medical instrument according to Appendix 1,

in which at least a part of a surface of the first region is formed as a curved surface, and
at least a part of a surface of the second region is formed as a curved surface.

Appendix 3

The medical instrument according to Appendix 1 or 2,
in which at least one of the first marker or the second marker includes a pattern row composed of an arrangement of the symbols not included in the other.

Appendix 4

The medical instrument according to Appendix 3,
in which the plurality of first pattern rows and the plurality of second pattern rows all have different arrangements of the symbols.

Appendix 5

The medical instrument according to Appendix 3 or 4,

in which at least one of the first marker or the second marker includes at least one pattern row including the symbol of a type not included in the other.

Appendix 6

The medical instrument according to any one of Appendices 1 to 5,
in which, in a case in which a direction from the first marker to the second marker is defined as a forward direction and a direction from the second marker to the first marker is defined as a reverse direction,

an arrangement of the symbols along the forward direction in at least one of the first pattern rows is the same as an arrangement of the symbols along the reverse direction in any one of the other first pattern rows, and
an arrangement of the symbols along the forward direction in at least one of the second pattern rows is the same as an arrangement of the symbols along the reverse direction in any one of the other second pattern rows.

Appendix 7

The medical instrument according to any one of Appendices 1 to 6,

in which, in the first marker, the plurality of first pattern rows are arranged such that only a combination of the first pattern rows having a Hamming distance of 2 or more is included in a predetermined range in the direction intersecting the longitudinal direction, and
in the second marker, the plurality of second pattern rows are arranged such that only a combination of the second pattern rows having a Hamming distance of 2 or more is included in a predetermined range in the direction intersecting the longitudinal direction.

Appendix 8

The medical instrument according to any one of Appendices 1 to 7,
in which types of the symbols constituting the first pattern row and types of the symbols constituting the second pattern row are the same.

Appendix 9

The medical instrument according to any one of Appendices 1 to 8,
in which colors of the symbols constituting the first pattern row and colors of the symbols constituting the second pattern row are all the same.

Appendix 10

The medical instrument according to any one of Appendices 1 to 9,
in which the number of the symbols constituting the first pattern row and the number of the symbols constituting the second pattern row are the same.

Appendix 11

The medical instrument according to any one of Appendices 1 to 10,

in which the first pattern row is configured by arranging a total of four symbols formed of two types of the symbols having different shapes, and
the second pattern row is configured by arranging a total of four symbols formed of the same two types of the symbols as the types of the symbols included in the first pattern row.

Appendix 12

The medical instrument according to any one of Appendices 1 to 11,
in which the intermediate region is provided with information other than the marker.

Appendix 13

The medical instrument according to Appendix 12,
in which the intermediate region is provided with information indicating a predetermined direction.

Appendix 14

The medical instrument according to Appendix 12 or 13,
in which the intermediate region is provided with gradations.

Appendix 15

The medical instrument according to Appendix 14,
in which the intermediate region is further provided with information indicating a center of the gradations.

Appendix 16

The medical instrument according to any one of Appendices 1 to 15,
in which the medical instrument is a medical probe capable of observing an internal structure of an organ.

Appendix 17

A medical support device comprising a processor, in which:

the medical support device specifies a position and an orientation of a medical instrument inserted into a body,
the medical instrument includes a first region provided with a first marker, an intermediate region not provided with a marker, and a second region provided with a second marker, which are disposed in a longitudinal direction,
the first marker includes plural first pattern rows arranged along a direction that intersects with the longitudinal direction, each of the first pattern rows being configured by plural symbols arranged along the longitudinal direction, an arrangement of the symbols in each first pattern row being different from an arrangement of the symbols in any first pattern row adjacent thereto,
the second marker includes plural second pattern rows arranged along the direction that intersects with the longitudinal direction, each of the second pattern rows being configured by plural symbols arranged along the longitudinal direction, an arrangement of the symbols in each second pattern row being different from an arrangement of the symbols in any second pattern row adjacent thereto, and
the processor is configured to:

acquire an optical image by optically imaging the medical instrument inserted into the body with a camera,
extract at least a part of pattern rows, from among the plurality of first pattern rows and the plurality of second pattern rows, from the optical image, and
specify the position and the orientation of the medical instrument based on at least the part of the pattern rows that is extracted.

Appendix 18

The medical support device according to Appendix 17,
in which the processor is configured to

specify a positional relationship between the first marker and the second marker based on a shape of the medical instrument included in the optical image, and
specify the position and the orientation of the medical instrument based on at least the part of the pattern rows that is extracted and the specified positional relationship between the first marker and the second marker.

Appendix 19

The medical support device according to Appendix 17 or 18,
in which the processor is configured to

detect the first region and the second region from the optical image using a trained model that has been trained in advance by using the optical image as an input and the first region and the second region in the input optical image as an output,

specify a positional relationship between the first marker and the second marker based on the detected first region and the detected second region, and

specify the position and the orientation of the medical instrument based on at least the part of the pattern rows that is extracted and the specified positional relationship between the first marker and the second marker.

Appendix 20

A medical support program for causing a computer to execute a process of specifying a position and an orientation of a medical instrument inserted into a body, in which:

the medical instrument includes a first region provided with a first marker, an intermediate region not provided with a marker, and a second region provided with a second marker, which are disposed in a longitudinal direction,

the first marker includes plural first pattern rows arranged along a direction that intersects with the longitudinal direction, each of the first pattern rows being configured by plural symbols arranged along the longitudinal direction, an arrangement of the symbols in each first pattern row being different from an arrangement of the symbols in any first pattern row adjacent thereto,

the second marker includes plural second pattern rows arranged along the direction that intersects with the longitudinal direction, each of the second pattern rows being configured by plural symbols arranged along the longitudinal direction, an arrangement of the symbols in each second pattern row being different from an arrangement of the symbols in any second pattern row adjacent thereto, and

the process comprising:

acquiring an optical image by optically imaging the medical instrument inserted into the body with a camera;

extracting at least a part of pattern rows, from among the plurality of first pattern rows and the plurality of second pattern rows, from the optical image; and

specifying the position and the orientation of the medical instrument based on at least the part of the pattern rows that is extracted.

## Claims

1. A medical instrument comprising a first region provided with a first marker, an intermediate region not provided with a marker, and a second region provided with a second marker, which are disposed in a longitudinal direction, wherein:

the first marker includes a plurality of first pattern rows arranged along a direction that intersects with the longitudinal direction, each of the first pattern rows being configured by a plurality of symbols arranged along the longitudinal direction, an arrangement of the symbols in each first pattern row being different from an arrangement of the symbols in any first pattern row adjacent thereto, and

the second marker includes a plurality of second pattern rows arranged along the direction that intersects with the longitudinal direction, each of the second pattern rows being configured by a plurality of symbols arranged along the longitudinal direction, an arrangement of the symbols in each second pattern row being different from an arrangement of the symbols in any second pattern row adjacent thereto.

2. The medical instrument according to claim 1, wherein:

at least a part of a surface of the first region is formed as a curved surface, and

at least a part of a surface of the second region is formed as a curved surface.

3. The medical instrument according to claim 1 or 2, wherein at least one of the first marker or the second marker includes a pattern row composed of an arrangement of the symbols not included in the other.

4. The medical instrument according to claim 3, wherein the plurality of first pattern rows and the plurality of second pattern rows all have different arrangements of the symbols.

5. The medical instrument according to claim 3 or 4, wherein at least one of the first marker or the second marker includes at least one pattern row including the symbol of a type not included in the other.

6. The medical instrument according to any one of claims 1 to 5, wherein, in a case in which a direction from the first marker to the second marker is defined as a forward direction and a direction from the second marker to the first marker is defined as a reverse direction:

an arrangement of the symbols along the forward direction in at least one of the first pattern rows is the same as an arrangement of the symbols along the reverse direction in any one of the other first pattern rows, and
an arrangement of the symbols along the forward direction in at least one of the second pattern rows is the same as an arrangement of the symbols along the reverse direction in any one of the other second pattern rows.

7. The medical instrument according to any one of claims 1 to 6, wherein:

in the first marker, the plurality of first pattern rows are arranged such that only a combination of the first pattern rows having a Hamming distance of 2 or more is included in a predetermined range in the direction intersecting the longitudinal direction, and
in the second marker, the plurality of second pattern rows are arranged such that only a combination of the second pattern rows having a Hamming distance of 2 or more is included in a predetermined range in the direction intersecting the longitudinal direction.

8. The medical instrument according to any one of claims 1 to 7, wherein:

types of the symbols constituting the first pattern row and types of the symbols constituting the second pattern row are the same;
colors of the symbols constituting the first pattern row and colors of the symbols constituting the second pattern row are all the same; or
the number of the symbols constituting the first pattern row and the number of the symbols constituting the second pattern row are the same.

9. The medical instrument according to any one of claims 1 to 8, wherein:

the first pattern row is configured by arranging a total of four symbols formed of two types of the symbols having different shapes, and
the second pattern row is configured by arranging a total of four symbols formed of the same two types of the symbols as the types of the symbols included in the first pattern row.

10. The medical instrument according to any one of claims 1 to 9, wherein the intermediate region is provided with information other than the marker, for example:

the intermediate region is provided with information indicating a predetermined direction; or
the intermediate region is provided with gradations, preferably further provided with information indicating a center of the gradations.

11. The medical instrument according to any one of claims 1 to 10, wherein the medical instrument is a medical probe capable of observing an internal structure of an organ.

12. A medical support device comprising a processor, wherein:

the medical support device specifies a position and an orientation of a medical instrument inserted into a body,
the medical instrument includes a first region provided with a first marker, an intermediate region not provided with a marker, and a second region provided with a second marker, which are disposed in a longitudinal direction,
the first marker includes a plurality of first pattern rows arranged along a direction that intersects with the longitudinal direction, each of the first pattern rows being configured by a plurality of symbols arranged along the longitudinal direction, an arrangement of the symbols in each first pattern row being different from an arrangement of the symbols in any first pattern row adjacent thereto, and
the second marker includes a plurality of second pattern rows arranged along the direction that intersects with the longitudinal direction, each of the second pattern rows being configured by a plurality of symbols arranged along the longitudinal direction, an arrangement of the symbols in each second pattern row being different from an arrangement of the symbols in any second pattern row adjacent thereto, and
the processor is configured to:

acquire an optical image by optically imaging the medical instrument inserted into the body with a camera;
extract at least a part of pattern rows, from among the plurality of first pattern rows and the plurality of second pattern rows, from the optical image; and
specify the position and the orientation of the medical instrument based on at least the part of the pattern rows that is extracted.

13. The medical support device according to claim 12, wherein the processor is configured to:

specify a positional relationship between the first marker and the second marker based on a shape of the medical instrument included in the optical image; and
specify the position and the orientation of the medical instrument based on at least the part of the pattern rows that is extracted and the specified positional relationship between the first marker and the second marker.

14. The medical support device according to claim 12 or 13, wherein the processor is configured to:

detect the first region and the second region from the optical image using a trained model that has been trained in advance by using the optical image as an input and the first region and the second region in the input optical image as an output;
specify a positional relationship between the first marker and the second marker based on the detected first region and the detected second region; and
specify the position and the orientation of the medical instrument based on at least the part of the pattern rows that is extracted and the specified positional relationship between the first marker and the second marker.

15. A computer-readable storage medium storing a medical support program that causes a computer to execute a process of specifying a position and an orientation of a medical instrument inserted into a body, in which:

the medical instrument includes a first region provided with a first marker, an intermediate region not provided with a marker, and a second region provided with a second marker, which are disposed in a longitudinal direction, the first marker includes a plurality of first pattern rows arranged along a direction that intersects with the longitudinal direction, each of the first pattern rows being configured by a plurality of symbols arranged along the longitudinal direction, an arrangement of the symbols in each first pattern row being different from an arrangement of the symbols in any first pattern row adjacent thereto, and
the second marker includes a plurality of second pattern rows arranged along the direction that intersects with the longitudinal direction, each of the second pattern rows being configured by a plurality of symbols arranged along the longitudinal direction, an arrangement of the symbols in each second pattern row being different from an arrangement of the symbols in any second pattern row adjacent thereto, and
the process comprising:

acquiring an optical image by optically imaging the medical instrument inserted into the body with a camera;
extracting at least a part of pattern rows, from among the plurality of first pattern rows and the plurality of second pattern rows, from the optical image; and
specifying the position and the orientation of the medical instrument based on at least the part of the pattern rows that is extracted.

FIG. 1

FIG. 2

## FIG. 3

## FIG. 4

FIG. 5

FIG. 6

FIG. 7

EP 4 652 957 A1

FIG. 8

# FIG. 9

11

| 41 — PROCESSOR | | | 48 | | | 43 RAM |
|---|---|---|---|---|---|---|

**PROCESSOR** (41)
- IMAGE ACQUISITION UNIT (41A)
- EXTRACTION UNIT (41B)
- SPECIFYING UNIT (41C)
- DISPLAY CONTROL UNIT (41D)

RAM — 43

**STORAGE** — 44
- MEDICAL SUPPORT PROGRAM — 49
- DIMENSION INFORMATION — 50

DISPLAY — 16

RECEPTION DEVICE — 42

COMMUNICATION I/F — 45

EXTERNAL I/F — 46

48

# FIG. 10

## FIG. 11

# FIG. 12

```
┌─────────────────────────────────┐
│   MEDICAL SUPPORT PROCESSING    │
└─────────────────────────────────┘
                 │
┌─────────────────────────────────┐
│      ACQUIRE OPTICAL IMAGE       │ ~S10
└─────────────────────────────────┘
                 │
┌─────────────────────────────────┐
│       EXTRACT PATTERN ROW        │ ~S12
└─────────────────────────────────┘
                 │
┌─────────────────────────────────┐
│  SPECIFY POSITION AND ORIENTATION│ ~S14
│      OF MEDICAL INSTRUMENT       │
└─────────────────────────────────┘
                 │
┌─────────────────────────────────┐
│    SPECIFY SUPPORT INFORMATION   │ ~S16
└─────────────────────────────────┘
                 │
┌─────────────────────────────────┐
│    PRESENT SUPPORT INFORMATION   │ ~S18
└─────────────────────────────────┘
                 │
┌─────────────────────────────────┐
│               END                │
└─────────────────────────────────┘
```

FIG. 13

64    74

FIG. 14

66    76

FIG. 15

64    74

FIG. 16

66    76

## FIG. 17

## FIG. 18

FIG. 19

FUJI123

EP 4 652 957 A1

**Europäisches Patentamt**
**European Patent Office**
**Office européen des brevets**

# EUROPEAN SEARCH REPORT

Application Number

EP 25 17 7372

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/168562 A1 (ZHAO TAO [US] ET AL) 1 July 2010 (2010-07-01) | 1-9, 12-15 | INV. A61B90/00 |
| Y | * paragraph [0109]; figures 13a-13c,21a,21b,22a-22d * | 10,11 | A61B34/20 |
| | ----- | | |
| Y | US 2020/193622 A1 (STOPP SEBASTIAN [DE] ET AL) 18 June 2020 (2020-06-18) * paragraph [0233] * | 10 | |
| | ----- | | |
| Y | WO 2023/162657 A1 (FUJIFILM CORP [JP]) 31 August 2023 (2023-08-31) * figure 9 * | 11 | |
| | ----- | | |
| X | WO 2009/085807 A1 (ST JUDE MEDICAL ATRIAL FIBRILL [US]; OLSON ERIC S [US] ET AL.) 9 July 2009 (2009-07-09) * figures 2,3 * | 1 | |
| | ----- | | |
| X | EP 3 031 494 A1 (NUCLETRON OPERATIONS BV [NL]) 15 June 2016 (2016-06-15) * paragraph [0034]; figure 2 * | 1 | **TECHNICAL FIELDS SEARCHED (IPC)** |
| | ----- | | A61B |

The present search report has been drawn up for all claims

| Place of search | Date of completion of the search | Examiner |
|---|---|---|
| Munich | 30 September 2025 | Franz, Volker |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 17 7372

This annex lists the patent family members relating to the patent documents cited in the above-mentioned European search report.
The members are as contained in the European Patent Office EDP file on
The European Patent Office is in no way liable for these particulars which are merely given for the purpose of information.

30-09-2025

| Patent document cited in search report | | Publication date | Patent family member(s) | | Publication date |
|---|---|---|---|---|---|
| US 2010168562 | A1 | 01-07-2010 | CN | 102341055 A | 01-02-2012 |
| | | | EP | 2391290 A1 | 07-12-2011 |
| | | | KR | 20110118639 A | 31-10-2011 |
| | | | US | 2010168562 A1 | 01-07-2010 |
| | | | WO | 2010078009 A1 | 08-07-2010 |
| US 2020193622 | A1 | 18-06-2020 | CA | 3005502 A1 | 21-09-2017 |
| | | | CA | 3112726 A1 | 21-09-2017 |
| | | | EP | 3429498 A1 | 23-01-2019 |
| | | | EP | 3735929 A1 | 11-11-2020 |
| | | | US | 2020193622 A1 | 18-06-2020 |
| | | | WO | 2017157763 A1 | 21-09-2017 |
| WO 2023162657 | A1 | 31-08-2023 | EP | 4487785 A1 | 08-01-2025 |
| | | | JP | WO2023162657 A1 | 31-08-2023 |
| | | | US | 2024407755 A1 | 12-12-2024 |
| | | | WO | 2023162657 A1 | 31-08-2023 |
| WO 2009085807 | A1 | 09-07-2009 | US | 2009171196 A1 | 02-07-2009 |
| | | | WO | 2009085807 A1 | 09-07-2009 |
| EP 3031494 | A1 | 15-06-2016 | CN | 105749431 A | 13-07-2016 |
| | | | EP | 3031494 A1 | 15-06-2016 |
| | | | ES | 2686469 T3 | 18-10-2018 |
| | | | US | 2016166328 A1 | 16-06-2016 |

EPO FORM P0459

For more details about this annex : see Official Journal of the European Patent Office, No. 12/82

**EP 4 652 957 A1**

**Patent documents cited in the description**

- JP 2018094020 A
- WO 2023162657 A
- JP 2017501802 A